# EUROPEAN PATENT APPLICATION

(11) **EP 4 292 609 A1**
(43) Date of publication of application: **20.12.2023**
(21) Application number: 22179260.9
(22) Date of filing: 15.06.2022
(51) Int. Cl.: A61K 39/395, A61K 31/70, C07K 16/28, C07K 16/46

(54) **COMPOSITIONS COMPRISING ANTIBODIES THAT BIND GAMMA-DELTA T CELL RECEPTORS**

(71) Applicant: LAVA Therapeutics N.V., 3584 CM Utrecht (NL)
(72) Inventor: PARREN, Paul Willem Henri Ida, 3584 CM Utrecht (NL); ROOVERS, Robertus Cornelis, 3584 CM Utrecht (NL); VAN DER VLIET, Johannes Jelle, 3584 CM Utrecht (NL); LUTJE HULSIK, David, 3584 CM Utrecht (NL); MACHIELSEN, Peter Alexander Gerardus Maria, 3584 CM Utrecht (NL); VAN WESTERHOVEN, Michiel, 3584 CM Utrecht (NL); KING, Lisa Anna, 3584 CM Utrecht (NL); FENNEMANN, Felix-Lennart, 3584 CM Utrecht (NL); VAN DER VEEN, Jochem Willem, 3584 CM Utrecht (NL)
(74) Representative: Cooley (UK) LLP

(57) **Abstract**

The present invention relates to pharmaceutical compositions comprising antibodies capable of binding a human Vγ9Vδ2 T cell receptor. The invention further relates to uses of the pharmaceutical compositions of the invention for medical treatment.

## Description

### Field of the invention

The present invention relates to novel pharmaceutical compositions comprising antibodies capable of binding the Vδ2 chain of a human Vγ9Vδ2 T cell receptor. The invention further relates to uses of the pharmaceutical compositions of the invention for medical treatment.

### Background of the invention

Gamma-delta (γδ) T cells are T cells that express a T cell receptor (TCR) that is made up of a gamma chain and a delta chain. The majority of γδ T cells in circulation express TCRs comprising Vγ9 and Vδ2 regions. Vγ9Vδ2 T cells can react against a wide array of pathogens and tumor cells. This broad reactivity is understood to be conferred by phosphoantigens that are able to specifically activate this T-cell subset in a TCR dependent fashion. The broad antimicrobial and antitumor reactivity of Vγ9Vδ2 T-cells suggest a direct involvement in immune control of cancers and infections.

Agents that can activate Vγ9Vδ2 T cells can be useful in the treatment of infections or cancer as these may promote Vγ9Vδ2 T cell reactivity towards the pathogen or infected cells or cancer cell. WO2015156673 describes antibodies that bind Vγ9Vδ2 TCRs and are capable of activating Vγ9Vδ2 T cells. WO2020060405 describes bispecific antibodies that bind both Vγ9Vδ2 T cells and a tumor cell target and thus have the potential to recruit Vγ9Vδ2 T cells to a tumor and thus stimulate a therapeutic effect.

Recombinant production of antibodies in host cells often results in heterogenous products, comprising different forms of the antibody with various types and degrees of post-translational modifications of the polypeptide chain. Such heterogeneity is undesirable for an antibody product for medical use, as post-translational modifications may alter the functional properties of the antibodies, for example in terms of affinity for the target antigen, in terms of pharmacokinetic properties, product stability, aggregation, etc.

The present invention provides pharmaceutical compositions comprising antibodies having improved Vγ9Vδ2 TCR binding antibody sequences that result in a more homogeneous product upon production in a host cell, yet retain good functional properties, with respect to target binding and functional effects on target cells, as well as good structural properties such as stability.

### Summary of the invention

PCT/EP2021/085079 (unpublished) describes that antibody 5C8 described in WO2015156673 undergoes a sulfation at a site in the antibody that was not predicted to be subject to this post-translational modification. Sulfation occurred partially in various host cells, resulting in a heterogenous antibody product. Surprisingly, the tyrosine residue subject to the sulfation could be mutated to a phenylalanine or a serine without affecting the antigen-binding properties of the antibody even though the amino acid is located in the CDR3 region, which is known to be the main determinant of antigen binding specificity in an antigen-binding region of an antibody. The removal of the sulfation site via mutation resulted in a more homogeneous antibody product.

The present invention provides novel pharmaceutical compositions comprising said mutated antibodies. Accordingly, in a first aspect, the invention provides a pharmaceutical composition comprising:
a. an antibody comprising a first antigen-binding region capable of binding to human Vδ2, wherein said first antigen-binding region comprises a CDR1 sequence as set forth in SEQ ID NO:1, a CDR2 sequence as set forth in SEQ ID NO:2 and a CDR3 sequence as set forth in SEQ ID NO:3,
b. 5-20 mM of a L-histidine buffer, wherein the composition has a pH between 5.5 and 6.5, or
   5-20 mM of a sodium acetate buffer, wherein the composition has a pH between 5.0 and 6.0,
c. 250-350 mM sucrose,
d. 0.01% - 0.05% (w/v) polysorbate 80, and
e. 0-20 mM L-methionine.

In a further aspect, the invention provides pharmaceutical compositions comprising bispecific antibodies comprising a first binding region capable of binding to human Vδ2 as defined herein and a second antigen-binding region capable of binding a second antigen, wherein the second antigen preferably is human EGFR.

In further aspects, the invention relates to uses of the pharmaceutical compositions comprising of the invention in medical treatment.

Further aspects and embodiments of the invention are described below.

### Brief description of the drawings

Figure 1: Representative chromatogram of the size exclusion profile of protein-A purified LAVA compound (VHH 5C8 is shown) using a Superdex-75 column. The fractions of the dominant monomeric peak (fractions 1E11-1G2) were pooled and quantified.
Figure 2: Representative example of labchip polyacrylamide gel electrophoresis of purified VHH 5C8. Left: non-reducing; right: reducing conditions.
Figure 3: HP-SEC profiles of purified VHH 5C8 (A) and VHH 5C8var1 (B).
Figure 4: Representative HP-SEC profiles of purified bispecific VHH (bsVHH) lD12var5-5C8varl. A: bsVHH 1D12var5-5C8var1 batch that was expressed and purified by protein-A affinity chromatography from the supernatant of a *Pichia pastoris* culture. B: bsVHH 1D12var5-5C8var1 batch that was expressed and purified by both protein-A and size exclusion chromatography from HEK-293 E cells.
Figure 5: Labchip analysis of purified VHH 5C8var1-Y105F and 5C8var1-Y105S under non-reducing conditions.
Figure 6: HP-SEC analysis of VHH 5C8var1-Y105F (A) and 5C8var1-Y105S (B).
Figure 7: Affinity measurement of VHH fragment binding to recombinant Vγ9Vδ2-TCR protein using BLI. The protein mass (response, in nm) is plotted as a function of time. The dotted vertical line separates the association phase (left) from the dissociation phase (right). A: VHH 5C8var1; B: VHH 5C8var1-Y105F; C: VHH 5C8var1-Y105S. Straight black lines represent fitted data to the actual responses measured.
Figure 8: Both bsVHH 7D12var8-5C8var1-Y105F and bsVHH 7D12-5C8 induce potent Vγ9Vδ2 T cell activation and cause Vγ9Vδ2 T cell-mediated tumour cell lysis. A: 4 hours degranulation assay: the percentage of CD107A (LAMP-1)+ Vγ9Vδ2 T cells is plotted as a function of the antibody concentration used. Left: 7D12-5C8 (non-humanised); right: 7D12var8-5C8var1-Y105F. B: 24 hours cytotoxicity assay showing the percentage of A431 tumour cell kill as a function of the antibody concentration used. Left: 7D12-5C8 (non-humanised); right: 7D12var8-5C8var1-Y105F.
Figure 9: Binding of 7D12var8-5C8var1(Y105F)-Fc to primary γδ T cells isolated from healthy human PBMCs using flow cytometry. The two panels represent two different donors.
Figure 10: Binding of 7D12var8-5C8var1(Y105F)-Fc to EGFR on tumor cells by cell-based ELISA.
Figure 11: Degranulation of γδ T cells induced by 7D12var8-5C8var1(Y105F)-Fc dependent on the A431 cell line.
Figure 12: Viability of A-388 cells in co-culture with γδ T cells and 7D12-5C8.
Figure 13: Lysed tumor cells after a 4-hour culture of dissociated tumor cell suspensions (primary CRC: n=10, peritoneal CRC metastases: n=5, liver CRC metastases: n=3, primary HNSCC: n=5, and primary NSCLC: n=4) with healthy donor derived Vγ9Vδ2 T cells (1:1 E:T ratio) and 7D12-5C8 (50 nM) or medium control.
Figure 14: Lysed tumor cells after a 24-hour culture of dissociated tumor cell suspensions (peritoneal CRC metastases: n=4) with healthy donor derived Vγ9Vδ2 T cells (1:1 E:T ratio) and 7D12-5C8var1(Y105S)-Fc (50 nM), gp120-5C8var1(Y105S)-Fc (50 nM) or medium control.
Figure 15: Structure of construct for non-human primate studies.
Figure 16: Binding of 7A5-7D12var8-Fc to antigen targets.
Figure 17: Degranulation and cytotoxicity mediated by 7A5-7D12var8-Fc.
Figure 18: PK analyses of 7A5-7D12var8-Fc concentrations in the blood of the three treated animals. Concentration-time curves are shown that demonstrate the molecule to have an IgG-like PK.
Figure 19: Total number of T cells (CD3+, left graph) and number of Vy9 positive cells (as percentage of the CD3+population) in the blood of treated animals. Arrows indicate the injections with compound. The numbers in the legend are the numbers of the treated monkeys.
Figure 20: Levels of the IL-6 cytokine in the blood of the treated animals over time. Only low levels of the cytokine were observed and the release was largely limited to after the first injection. Arrowheads indicate the treatment moments.

### Detailed description of the invention

### Definitions

The term "human Vδ2", when used herein, refers to the rearranged δ2 chain of the Vγ9Vδ2-T cell receptor (TCR). UniProtKB - A0JD36 (A0JD36_HUMAN) gives an example of a variable TRDV2 sequence. Vδ2 is part of the delta chain of the Vγ9Vδ2-TCR. An antibody capable of binding to human Vδ2 may bind an epitope that is entirely located within the variable region or bind an epitope that is located within the constant region or bind an epitope that is a combination of residues of the variable and constant regions of the delta chain.

The term "human Vy9", when used herein, refers to the rearranged y9 chain of the Vγ9Vδ2-T cell receptor (TCR). UniProtKB - Q99603_HUMAN gives an example of a variable TRGV9 sequence.

The term "EGFR", when used herein, refers to the human EGFR protein (UniProtKB - P00533 (EGFR_HUMAN)).

The term "antibody" is intended to refer to an immunoglobulin molecule, a fragment of an immunoglobulin molecule, or a derivative of either thereof, which has the ability to specifically bind to an antigen under typical physiological conditions with a half-life of significant periods of time, such as at least about 30 minutes, at least about 45 minutes, at least about one hour, at least about two hours, at least about four hours, at least about 8 hours, at least about 12 hours, about 24 hours or more, about 48 hours or more, about 3, 4, 5, 6, 7 or more days, etc., or any other relevant functionally-defined period (such as a time sufficient to induce, promote, enhance, and/or modulate a physiological response associated with antibody binding to the antigen and/or time sufficient for the antibody to recruit an effector activity). The antigen-binding region (or antigen-binding domain) which interacts with an antigen may comprise variable regions of both the heavy and light chains of the immunoglobulin molecule or may be a single-domain antigen-binding region, e.g. a heavy chain variable region only. The constant regions of an antibody, if present, may mediate the binding of the immunoglobulin to host tissues or factors, including various cells of the immune system (such as effector cells and T cells) and components of the complement system such as C1q, the first component in the classical pathway of complement activation.

The Fc region of an immunoglobulin is defined as the fragment of an antibody which would be typically generated after digestion of an antibody with papain which includes the two CH2-CH3 regions of an immunoglobulin and a connecting region, e.g. a hinge region. The constant domain of an antibody heavy chain defines the antibody isotype, e.g. IgG1, IgG2, IgG3, IgG4, IgA1, IgA2, IgM, IgD, or IgE. The Fc-region mediates the effector functions of antibodies with cell surface receptors called Fc receptors and proteins of the complement system.

The term "hinge region" as used herein is intended to refer to the hinge region of an immunoglobulin heavy chain. Thus, for example, the hinge region of a human IgG1 antibody corresponds to amino acids 216-230 according to the EU numbering.

The term "CH2 region" or "CH2 domain" as used herein is intended to refer to the CH2 region of an immunoglobulin heavy chain. Thus, for example the CH2 region of a human IgG1 antibody corresponds to amino acids 231-340 according to the EU numbering. However, the CH2 region may also be any of the other subtypes as described herein.

The term "CH3 region" or "CH3 domain" as used herein is intended to refer to the CH3 region of an immunoglobulin heavy chain. Thus, for example the CH3 region of a human IgG1 antibody corresponds to amino acids 341-447 according to the EU numbering. However, the CH3 region may also be any of the other subtypes as described herein.

Reference to amino acid positions in the Fc region/Fc domain in the present invention is according to the EU-numbering (Edelman et al., Proc Natl Acad Sci U S A. 1969 May;63(1):78-85; Kabat et al., Sequences of proteins of immunological interest. 5th Edition - 1991 NIH Publication No. 91-3242).

As indicated above, the term antibody as used herein, unless otherwise stated or clearly contradicted by context, includes fragments of an antibody that retain the ability to specifically bind to the antigen. It has been shown that the antigen-binding function of an antibody may be performed by fragments of a full-length antibody. Examples of binding fragments encompassed within the term "antibody" include (i) a Fab' or Fab fragment, i.e. a monovalent fragment consisting of the VL, VH, CL and CH1 domains, or a monovalent antibody as described in WO2007059782; (ii) F(ab')2 fragments, i.e. bivalent fragments comprising two Fab fragments linked by a disulfide bridge at the hinge region; (iii) a Fd fragment consisting essentially of the VH and CH1 domains; and (iv) a Fv fragment consisting essentially of the VL and VH domains of a single arm of an antibody. Furthermore, although the two domains of the Fv fragment, VL and VH, are coded for by separate genes, they may be joined, using recombinant methods, by a synthetic linker that enables them to be made as a single protein chain in which the VL and VH regions pair to form monovalent molecules (known as single chain antibodies or single chain Fv (scFv), see for instance Bird et al., Science 242, 423-426 (1988) and Huston et al., PNAS USA 85, 5879-5883 (1988)). Such single chain antibodies are encompassed within the term antibody unless otherwise indicated by context. Although such fragments are generally included within the meaning of antibody, they collectively and each independently are unique features of the present invention, exhibiting different biological properties and utility. The term antibody, unless specified otherwise, also includes polyclonal antibodies, monoclonal antibodies (mAbs), chimeric antibodies and humanized antibodies, and antibody fragments provided by any known technique, such as enzymatic cleavage, peptide synthesis, and recombinant techniques.

In some embodiments of the antibodies of the invention, the first antigen-binding region or the second antigen-binding region, or both, is a single domain antibody. Single domain antibodies are well known to the skilled person, see e.g. Hamers-Casterman et al. (1993) Nature 363:446, Roovers et al. (2007) Curr Opin Mol Ther 9:327 and Krah et al. (2016) Immunopharmacol Immunotoxicol 38:21. Single domain antibodies comprise a single CDR1, a single CDR2 and a single CDR3. Examples of single domain antibodies are variable fragments of heavy-chain-only antibodies, antibodies that naturally do not comprise light chains, single domain antibodies derived from conventional antibodies, and engineered antibodies. Single domain antibodies may be derived from any species including mouse, human, camel, llama, shark, goat, rabbit, and cow. For example, single domain antibodies can be derived from antibodies raised in Camelidae species, for example in camel, dromedary, llama, alpaca and guanaco. Like a whole antibody, a single domain antibody is able to bind selectively to a specific antigen. Single domain antibodies may contain only the variable domain of an immunoglobulin chain, i.e. CDR1, CDR2 and CDR3 and framework regions. Such antibodies are also called Nanobody^{®}, or VHH.

The term "immunoglobulin" as used herein is intended to refer to a class of structurally related glycoproteins consisting of two pairs of polypeptide chains, one pair of light (L) chains and one pair of heavy (H) chains, all four potentially inter-connected by disulfide bonds. The term "immunoglobulin heavy chain", "heavy chain of an immunoglobulin" or "heavy chain" as used herein is intended to refer to one of the chains of an immunoglobulin. A heavy chain is typically comprised of a heavy chain variable region (abbreviated herein as VH) and a heavy chain constant region (abbreviated herein as CH) which defines the isotype of the immunoglobulin. The heavy chain constant region typically is comprised of three domains, CH1, CH2, and CH3. The heavy chain constant region further comprises a hinge region. Within the structure of the immunoglobulin (e.g. IgG), the two heavy chains are inter-connected via disulfide bonds in the hinge region. Equally to the heavy chains, each light chain is typically comprised of several regions; a light chain variable region (VL) and a light chain constant region (CL). Furthermore, the VH and VL regions may be subdivided into regions of hypervariability (or hypervariable regions which may be hypervariable in sequence and/or form structurally defined loops), also termed complementarity determining regions (CDRs), interspersed with regions that are more conserved, termed framework regions (FRs). Each VH and VL is typically composed of three CDRs and four FRs, arranged from amino-terminus to carboxy-terminus in the following order: FR1, CDR1, FR2, CDR2, FR3, CDR3, FR4. CDR sequences may be determined by use of various methods, e.g. the methods provided by Chothia and Lesk (1987) J. Mol. Biol. 196:901 or Kabat et al. (1991) Sequence of protein of immunological interest, fifth edition. NIH publication. Various methods for CDR determination and amino acid numbering can be compared on www.abysis.org (UCL).

The term "isotype" as used herein, refers to the immunoglobulin (sub)class (for instance IgG1, IgG2, IgG3, IgG4, IgD, IgA, IgE, or IgM) or any allotype thereof, such as IgG1m(za) and IgG1m(f) that is encoded by heavy chain constant region genes. Each heavy chain isotype can be combined with either a kappa (κ) or lambda (λ) light chain. An antibody of the invention can possess any isotype.

The term "parent antibody", is to be understood as an antibody which is identical to an antibody according to the invention, but wherein the parent antibody does not have one or more of the specified mutations. A "variant" or "antibody variant" or a "variant of a parent antibody" of the present invention is an antibody molecule which comprises one or more mutations as compared to a "parent antibody". Amino acid substitutions may exchange a native amino acid for another naturally-occurring amino acid, or for a non-naturally-occurring amino acid derivative. The amino acid substitution may be conservative or non-conservative. In the context of the present invention, conservative substitutions may be defined by substitutions within the classes of amino acids reflected in one or more of the following three tables:

**Amino acid residue classes for conservative substitutions**

| | |
|---|---|
| Acidic Residues | Asp (D) and Glu (E) |
| Basic Residues | Lys (K), Arg (R), and His (H) |
| Hydrophilic Uncharged Residues | Ser (S), Thr (T), Asn (N), and Gln (Q) |
| Aliphatic Uncharged Residues | Gly (G), Ala (A), Val (V), Leu (L), and Ile (I) |
| Non-polar Uncharged Residues | Cys (C), Met (M), and Pro (P) |
| Aromatic Residues | Phe (F), Tyr (Y), and Trp (W) |

**Alternative conservative amino acid residue substitution classes**

| | | | |
|---|---|---|---|
| 1 | A | S | T |
| 2 | D | E | |
| 3 | N | Q | |
| 4 | R | K | |
| 5 | I | L | M |
| 6 | F | Y | W |

**Alternative Physical and Functional Classifications of Amino Acid Residues**

| | |
|---|---|
| Alcohol group-containing residues | S and T |
| Aliphatic residues | I, L, V, and M |
| Cycloalkenyl-associated residues | F, H, W, and Y |
| Hydrophobic residues | A, C, F, G, H, I, L, M, R, T, V, W, and Y |
| Negatively charged residues | D and E |
| Polar residues | C, D, E, H, K, N, Q, R, S, and T |
| Positively charged residues | H, K, and R |
| Small residues | A, C, D, G, N, P, S, T, and V |
| Very small residues | A, G, and S |
| Residues involved in turn formation | A, C, D, E, G, H, K, N, Q, R, S, P, and T |
| Flexible residues | Q, T, K, S, G, N, D, E, and R |

In the context of the present invention, a substitution in a variant is indicated as:
Original amino acid - position - substituted amino acid;

The three-letter code, or one letter code, are used, including the codes Xaa and X to indicate amino acid residue. Accordingly, the notation "T366W" means that the variant comprises a substitution of threonine with tryptophan in the variant amino acid position corresponding to the amino acid in position 366 in the parent antibody.

Furthermore, the term "a substitution" embraces a substitution into any one of the other nineteen natural amino acids, or into other amino acids, such as nonnatural amino acids. For example, a substitution of amino acid T in position 366 includes each of the following substitutions: 366A, 366C, 366D, 366G, 366H, 366F, 3661, 366K, 366L, 366M, 366N, 366P, 366Q, 366R, 366S, 366E, 366V, 366W, and 366Y.

The term "full-length antibody" when used herein, refers to an antibody which contains all heavy and light chain constant and variable domains corresponding to those that are normally found in a wild-type antibody of that isotype.

The term "chimeric antibody" refers to an antibody wherein the variable region is derived from a non-human species (e.g. derived from rodents) and the constant region is derived from a different species, such as human. Chimeric antibodies may be generated by genetic engineering. Chimeric monoclonal antibodies for therapeutic applications are developed to reduce antibody immunogenicity.

The term "humanized antibody" refers to a genetically engineered non-human antibody, which contains human antibody constant domains and non-human variable domains modified to contain a high level of sequence homology to human variable domains. This can be achieved by grafting of the six non-human antibody complementarity-determining regions (CDRs), which together form the antigen binding site, onto a homologous human acceptor framework region (FR). In order to fully reconstitute the binding affinity and specificity of the parental antibody, the substitution of framework residues from the parental antibody (i.e. the non-human antibody) into the human framework regions (back-mutations) may be required. Structural homology modeling may help to identify the amino acid residues in the framework regions that are important for the binding properties of the antibody. Thus, a humanized antibody may comprise non-human CDR sequences, primarily human framework regions optionally comprising one or more amino acid back-mutations to the non-human amino acid sequence, and, optionally, fully human constant regions. Optionally, additional amino acid modifications, which are not necessarily back-mutations, may be introduced to obtain a humanized antibody with preferred characteristics, such as affinity and biochemical properties. Humanization of non-human therapeutic antibodies is performed to minimize its immunogenicity in man while such humanized antibodies at the same time maintain the specificity and binding affinity of the antibody of non-human origin.

The term "multispecific antibody" refers to an antibody having specificities for at least two different, such as at least three, typically non-overlapping, epitopes. Such epitopes may be on the same or on different target antigens. If the epitopes are on different targets, such targets may be on the same cell or different cells or cell types. In some embodiments, a multispecific antibody may comprise one or more single-domain antibodies.

The term "bispecific antibody" refers to an antibody having specificities for two different, typically non-overlapping, epitopes. Such epitopes may be on the same or different targets. If the epitopes are on different targets, such targets may be on the same cell or different cells or cell types. In some embodiments, a bispecific antibody may comprise one or two single-domain antibodies.

Examples of different classes of multispecific, such as bispecific, antibodies include but are not limited to (i) IgG-like molecules with complementary CH3 domains to force heterodimerization; (ii) recombinant IgG-like dual targeting molecules, wherein the two sides of the molecule each contain the Fab fragment or part of the Fab fragment of at least two different antibodies; (iii) IgG fusion molecules, wherein full length IgG antibodies are fused to extra Fab fragment or parts of Fab fragment; (iv) Fc fusion molecules, wherein single chain Fv molecules or stabilized diabodies are fused to heavy-chain constant- domains, Fc-regions or parts thereof; (v) Fab fusion molecules, wherein different Fab- fragments are fused together, fused to heavy-chain constant-domains, Fc-regions or parts thereof; and (vi) ScFv-and diabody-based and heavy chain antibodies (e.g., domain antibodies, Nanobodies^{®}) wherein different single chain Fv molecules or different diabodies or different heavy-chain antibodies (e.g. domain antibodies, Nanobodies^{®}) are fused to each other or to another protein or carrier molecule fused to heavy-chain constant-domains, Fc-regions or parts thereof.

Examples of IgG-like molecules with complementary CH3 domains molecules include but are not limited to the Triomab^{®} (Trion Pharma/Fresenius Biotech), the Knobs-into-Holes (Genentech), CrossMAbs (Roche) and the electrostaticallymatched (Amgen, Chugai, Oncomed), the LUZ-Y (Genentech, Wranik et al. J. Biol. Chem. 2012, 287(52): 43331-9, doi: 10.1074/jbc.M112.397869. Epub 2012 Nov 1), DIG-body and PIG-body (Pharmabcine, WO2010134666, WO2014081202), the Strand Exchange Engineered Domain body (SEEDbody)(EMD Serono), the Biclonics (Merus, WO2013157953), FcΔAdp (Regeneron), bispecific IgG1 and IgG2 (Pfizer/Rinat), Azymetric scaffold (Zymeworks/Merck), mAb-Fv (Xencor), bivalent bispecific antibodies (Roche, WO2009080254) and DuoBody^{®} molecules (Genmab).

Examples of recombinant IgG-like dual targeting molecules include but are not limited to Dual Targeting (DT)-Ig (GSK/Domantis, WO2009058383), Two-in-one Antibody (Genentech, Bostrom, et al 2009. Science 323, 1610-1614), Cross-linked Mabs (Karmanos Cancer Center), mAb2 (F-Star), Zybodies^{™} (Zyngenia, LaFleur et al. MAbs. 2013 Mar-Apr;5(2):208-18), approaches with common light chain, κλBodies (NovImmune, WO2012023053) and CovX-body^{®} (CovX/Pfizer, Doppalapudi, V.R., et al 2007. Bioorg. Med. Chem. Lett. 17,501-506).

Examples of IgG fusion molecules include but are not limited to Dual Variable Domain (DVD)-Ig (Abbott), Dual domain double head antibodies (Unilever; Sanofi Aventis), IgG-like Bispecific (ImClone/Eli Lilly, Lewis et al. Nat Biotechnol. 2014 Feb;32(2): 191-8), Ts2Ab (MedImmune/AZ, Dimasi et al. J Mol Biol. 2009 Oct 30;393(3):672-92) and BsAb (Zymogenetics, WO2010111625), HERCULES (Biogen Idec), scFv fusion (Novartis), scFv fusion (Changzhou Adam Biotech Inc) and TvAb (Roche).

Examples of Fc fusion molecules include but are not limited to ScFv/Fc Fusions (Academic Institution, Pearce et al Biochem Mol Biol Int. 1997 Sep;42(6):1179), SCORPION (Emergent BioSolutions/Trubion, Blankenship JW, et al. AACR 100th Annual meeting 2009 (Abstract #5465); Zymogenetics/BMS, WO2010111625), Dual Affinity Retargeting Technology (Fc-DARTTM) (MacroGenics) and Dual(ScFv)2-Fab (National Research Center for Antibody Medicine - China).

Examples of Fab fusion bispecific antibodies include but are not limited to F(ab)2 (Medarex/AMGEN), Dual-Action or Bis-Fab (Genentech), Dock-and-Lock^{®} (DNL) (ImmunoMedics), Bivalent Bispecific (Biotecnol) and Fab-Fv (UCB-Celltech).

Examples of ScFv-, diabody-based and domain antibodies include but are not limited to Bispecific T Cell Engager (BiTE^{®}) (Micromet, Tandem Diabody (Tandab) (Affimed), Dual Affinity Retargeting Technology (DARTTM) (MacroGenics), Singlechain Diabody (Academic, Lawrence FEBS Lett. 1998 Apr 3;425(3):479-84), TCRlike Antibodies (AIT, ReceptorLogics), Human Serum Albumin ScFv Fusion (Merrimack, WO2010059315) and COMBODY molecules (Epigen Biotech, Zhu et al. Immunol Cell Biol. 2010 Aug;88(6):667-75), dual targeting nanobodies^{®} (Ablynx, Hmila et al., FASEB J. 2010), dual targeting heavy chain only domain antibodies.

In the context of antibody binding to an antigen, the terms "binds" or "specifically binds" refer to the binding of an antibody to a predetermined antigen or target (e.g. human Vδ2 or human EGFR) to which binding typically is with an apparent affinity corresponding to a K_{D} of about 10⁻⁶ M or less, e.g. 10⁻⁷ M or less, such as about 10⁻⁸ M or less, such as about 10⁻⁹ M or less, about 10⁻¹⁰ M or less, or about 10⁻¹¹ M or even less, e.g. when determined using flow cytometry as described in the Examples herein. Alternatively, K_{D} values can be determined using for instance surface plasmon resonance (SPR) technology in a BIAcore T200 or biolayer interferometry (BLI) in an Octet RED96 instrument using the antigen as the ligand and the binding moiety or binding molecule as the analyte. Specific binding means that the antibody binds to the predetermined antigen with an affinity corresponding to a K_{D} that is at least ten-fold lower, such as at least 100-fold lower, for instance at least 1,000-fold lower, such as at least 10,000-fold lower, for instance at least 100,000-fold lower than its affinity for binding to a non-specific antigen (e.g., BSA, casein) other than the predetermined antigen or a closelyrelated antigen. The degree with which the affinity is lower is dependent on the K_{D} of the binding moiety or binding molecule, so that when the K_{D} of the binding moiety or binding molecule is very low (that is, the binding moiety or binding molecule is highly specific), then the degree with which the affinity for the antigen is lower than the affinity for a non-specific antigen may be at least 10,000-fold. The term "K_{D}" (M), as used herein, refers to the dissociation equilibrium constant of a particular interaction between the antigen and the binding moiety or binding molecule.

In the context of the present invention, "competition" or "able to compete" or "competes" refers to any detectably significant reduction in the propensity for a particular binding molecule (e.g. an EGFR antibody) to bind a particular binding partner (e.g. EGFR) in the presence of another molecule (e.g. a different EGFR antibody) that binds the binding partner. Typically, competition means an at least about 25 percent reduction, such as an at least about 50 percent, e.g. an at least about 75 percent, such as an at least 90 percent reduction in binding, caused by the presence of another molecule, such as an antibody, as determined by, e.g., ELISA analysis or flow cytometry using sufficient amounts of the two or more competing molecules, e.g. antibodies. Additional methods for determining binding specificity by competitive inhibition may be found in for instance Harlow et al., Antibodies: A Laboratory Manual, Cold Spring Harbor Laboratory Press, Cold Spring Harbor, N.Y., 1988), Colligan et al., eds., Current Protocols in Immunology, Greene Publishing Assoc, and Wiley InterScience N. Y., (1992, 1993), and Muller, Meth. Enzymol. 92, 589-601 (1983)).

In one embodiment, the antibody of the present invention binds to the same epitope on EGFR as antibody 7D12 and/or to the same epitope on Vδ2 as antibody 5C8. There are several methods available for mapping antibody epitopes on target antigens known in the art, including but not limited to: crosslinking coupled mass spectrometry, allowing identification of peptides that are part of the epitope, and X-ray crystallography identifying individual residues on the antigen that form the epitope. Epitope residues can be determined as being all amino acid residues with at least one atom less than or equal to 5 Å from the antibody. 5 Å was chosen as the epitope cutoff distance to allow for atoms within a van der Waals radius plus a possible water-mediated hydrogen bond. Next, epitope residues can be determined as being all amino acid residues with at least one atom less than or equal to 8 Å. Less than or equal to 8 Å is chosen as the epitope cutoff distance to allow for the length of an extended arginine amino acid. Crosslinking coupled mass spectrometry begins by binding the antibody and the antigen with a mass labeled chemical crosslinker. Next the presence of the complex is confirmed using high mass MALDI detection. Because after crosslinking chemistry the Ab/Ag complex is extremely stable, many various enzymes and digestion conditions can be applied to the complex to provide many different overlapping peptides. Identification of these peptides is performed using high resolution mass spectrometry and MS/MS techniques. Identification of the crosslinked peptides is determined using mass tag linked to the cross-linking reagents. After MS/MS fragmentation and data analysis, peptides that are crosslinked and are derived from the antigen are part of the epitope, while peptides derived from the antibody are part of the paratope. All residues between the most N- and C-terminal crosslinked residue from the individual crosslinked peptides found are considered to be part of the epitope or paratope. The epitope of antibody 7D12 has been determined by X-ray crystallography, described in Schmitz et al. (2013) Structure 21:1214 and consists of a flat surface on domain III (residues R353, D355, F357, Q384, N420) that corresponds to the domain III ligand-binding site.

The terms "first" and "second" antigen-binding regions when used herein do not refer to their orientation / position in the antibody, i.e. they have no meaning with regard to the N- or C-terminus. The terms "first" and "second" only serve to correctly and consistently refer to the two different antigen-binding regions in the claims and the description.

"% sequence identity", when used herein, refers to the number of identical nucleotide or amino acid positions shared by different sequences (i.e., % identity = # of identical positions/total # of positions x 100), taking into account the number of gaps, and the length of each gap, which need to be introduced for optimal alignment. The percent identity between two nucleotide or amino acid sequences may e.g. be determined using the algorithm of E. Meyers and W. Miller, Comput. Appl. Biosci 4, 11-17 (1988) which has been incorporated into the ALIGN program (version 2.0), using a PAM120 weight residue table, a gap length penalty of 12 and a gap penalty of 4.

### Further aspects and embodiments of the invention

As described, in a first aspect, the invention relates to a pharmaceutical composition comprising:
a. an antibody comprising a first antigen-binding region capable of binding to human Vδ2, wherein said first antigen-binding region comprises a CDR1 sequence as set forth in SEQ ID NO:1, a CDR2 sequence as set forth in SEQ ID NO:2 and a CDR3 sequence as set forth in SEQ ID NO:3,
b. 5-20 mM of a L-histidine buffer, wherein the composition has a pH between 5.5 and 6.5, or
   5-20 mM of a sodium acetate buffer, wherein the composition has a pH between 5.0 and 6.0,
c. 250-350 mM sucrose,
d. 0.01% - 0.05% (w/v) polysorbate 80, and
e. 0-20 mM L-methionine.

In one embodiment, the composition comprises 5-20 mM, such as 10 mM, of a L-histidine buffer and has a pH between 5.5 and 6.5.

In another embodiment, the composition comprises 5-20 mM of a sodium acetate buffer and has a pH between 5.0 and 6.0.

In one embodiment, the composition comprises between 250 and 300 mM, such as 280 mM sucrose.

In one embodiment, the composition comprises between 0.01% and 0.03%, such as 0.02%, polysorbate 80.

In one embodiment, the composition comprises between 0.5 and 20 mM L-methionine, such as 1 mM of L-methionine.

In one embodiment, the composition comprises between 0.2 and 25 mg/mL, such as between 0.2 and 20 mg/mL, for example 1 or 10 mg/mL, of said antibody.

In one embodiment, the composition comprises:
- 5-20 mM, such as 10 mM, of a L-histidine buffer and has a pH between 5.5 and 6.5, and
- between 250 and 300 mM, such as 280 mM sucrose, and
- between 0.01% and 0.03%, such as 0.02%, polysorbate 80.

In one embodiment, the composition comprises:
- 10 mM of a L-histidine buffer and has a pH between 5.5 and 6.5, and
- 280 mM sucrose, and
- 0.02% polysorbate 80.

In one embodiment, the composition comprises:
- 5-20 mM, such as 10 mM, of a L-histidine buffer and has a pH between 5.5 and 6.5, and
- between 250 and 300 mM, such as 280 mM sucrose, and
- between 0.01% and 0.03%, such as 0.02%, polysorbate 80, and
- between 0.5 and 20 mM L-methionine, such as 1 mM of L-methionine.

In one embodiment, the composition comprises:
- 10 mM of a L-histidine buffer and has a pH between 5.5 and 6.5, and
- 280 mM sucrose, and
- 0.02% polysorbate 80, and
- 1 mM of L-methionine.

In one embodiment, the composition comprises between 0.2 and 20 mg/mL, such as 1 or 10 mg/mL, of said antibody.

In one embodiment, the composition comprises:
- 5-20 mM, such as 10 mM, of a L-histidine buffer and has a pH between 5.5 and 6.5, and
- between 250 and 300 mM, such as 280 mM sucrose, and
- between 0.01% and 0.03%, such as 0.02%, polysorbate 80, and
- 1 mg/mL of said antibody.

In one embodiment, the composition comprises:
- 10 mM of a L-histidine buffer and has a pH between 5.5 and 6.5, and
- 280 mM sucrose, and
- 0.02% polysorbate 80, and
- 1 mg/mL of said antibody.

In one embodiment, the composition comprises:
- 5-20 mM, such as 10 mM, of a L-histidine buffer and has a pH between 5.5 and 6.5, and
- between 250 and 300 mM, such as 280 mM sucrose, and
- between 0.01% and 0.03%, such as 0.02%, polysorbate 80, and
- between 0.5 and 20 mM L-methionine, such as 1 mM of L-methionine, and
- 1 mg/mL of said antibody.

In one embodiment, the composition comprises:
- 10 mM of a L-histidine buffer and has a pH between 5.5 and 6.5, and
- 280 mM sucrose, and
- 0.02% polysorbate 80, and
- 1 mM of L-methionine, and
- 1 mg/mL of said antibody.

In one embodiment, the composition comprises:
- 5-20 mM, such as 10 mM, of a L-histidine buffer and has a pH between 5.5 and 6.5, and
- between 250 and 300 mM, such as 280 mM sucrose, and
- between 0.01% and 0.03%, such as 0.02%, polysorbate 80, and
- 10 mg/mL of said antibody.

In one embodiment, the composition comprises:
- 10 mM of a L-histidine buffer and has a pH between 5.5 and 6.5, and
- 280 mM sucrose, and
- 0.02% polysorbate 80, and
- 10 mg/mL of said antibody.

In one embodiment, the composition comprises:
- 5-20 mM, such as 10 mM, of a L-histidine buffer and has a pH between 5.5 and 6.5, and
- between 250 and 300 mM, such as 280 mM sucrose, and
- between 0.01% and 0.03%, such as 0.02%, polysorbate 80, and
- between 0.5 and 20 mM L-methionine, such as 1 mM of L-methionine, and
- 10 mg/mL of said antibody.

In one embodiment, the composition comprises:
- 10 mM of a L-histidine buffer and has a pH between 5.5 and 6.5, and
- 280 mM sucrose, and
- 0.02% polysorbate 80, and
- 1 mM of L-methionine, and
- 10 mg/mL of said antibody.

In one embodiment, the composition comprises:
- 5-20 mM, such as 10 mM, of a L-histidine buffer and has a pH between 5.5 and 6.5, and
- between 250 and 300 mM, such as 280 mM sucrose, and
- between 0.01% and 0.03%, such as 0.02%, polysorbate 80, and
- 20 mg/mL of said antibody.

In one embodiment, the composition comprises:
- 10 mM of a L-histidine buffer and has a pH between 5.5 and 6.5, and
- 280 mM sucrose, and
- 0.02% polysorbate 80, and
- 20 mg/mL of said antibody.

In one embodiment, the composition comprises:
- 5-20 mM, such as 10 mM, of a L-histidine buffer and has a pH between 5.5 and 6.5, and
- between 250 and 300 mM, such as 280 mM sucrose, and
- between 0.01% and 0.03%, such as 0.02%, polysorbate 80, and
- between 0.5 and 20 mM L-methionine, such as 1 mM of L-methionine, and
- 20 mg/mL of said antibody.

In one embodiment, the composition comprises:
- 10 mM of a L-histidine buffer and has a pH between 5.5 and 6.5, and
- 280 mM sucrose, and
- 0.02% polysorbate 80, and
- 1 mM of L-methionine, and
- 20 mg/mL of said antibody.

As described above, in a first aspect, the invention relates to pharmaceutical composition comprising an antibody comprising a first antigen-binding region capable of binding to human Vδ2, wherein said first antigen-binding region comprises a CDR1 sequence as set forth in SEQ ID NO:1, a CDR2 sequence as set forth in SEQ ID NO:2 and a CDR3 sequence as set forth in SEQ ID NO:3.

In one embodiment, X₁ in SEQ ID NO:1 is S (Ser). In another embodiment, X₁ in SEQ ID NO:1 is G (Gly).

In one embodiment, X₂ in SEQ ID NO:3 is F (Phe). In another embodiment, X₂ in SEQ ID NO:3 is S (Ser).

In one embodiment, X₁ in SEQ ID NO:1 is S (Ser) and X₂ in SEQ ID NO:3 is F (Phe).

In one embodiment, X₁ in SEQ ID NO:1 is S (Ser) and X₂ in SEQ ID NO:3 is S (Ser).

In one embodiment, X₁ in SEQ ID NO:1 is G (Gly) and X₂ in SEQ ID NO:3 is F (Phe).

In one embodiment, X₁ in SEQ ID NO:1 is G (Gly) and X₂ in SEQ ID NO:3 is S (Ser).

In a preferred embodiment, the antibody is able to activate human Vγ9Vδ2 T cells. Activation of Vγ9Vδ2 T cells may be measured through measuring alterations in gene-expression and/or (surface) marker expression (e.g., activation markers, such as CD25, CD69, or CD107a) and/or secretory protein (e.g., cytokines or chemokines) profiles. In a preferred embodiment, the antibody is able to induce activation (e.g. upregulation of CD69 and/or CD25 expression) resulting in degranulation marked by an increase in CD107a expression and/or cytokine production (e.g. TNF, IFNγ) by Vγ9Vδ2 T cells.

In a further preferred embodiment, the antibody is able to increase the number of cells positive for CD107a at least 2-fold, such as at least 5-fold, when tested as described in Example 9 herein, e.g. at a concentration of 1nM, preferably 100pM, preferably 10pM, preferably 1pM, even more preferably 100fM. In another preferred embodiment, the antibody of the invention has an EC50 value for increasing the percentage of CD107a positive cells of 100 pM or less, such as 50 pM or less, e.g. 25 pM or less, such as 20 pM or less, e.g. 15 pM or less when tested using Vγ9Vδ2 T cells and A431 target cells as described herein in Example 9.

In one embodiment, the first antigen-binding region is a single-domain antibody. Thus, in one embodiment, the antibody in the pharmaceutical composition of the invention comprises a single-domain antibody capable of binding to human Vδ2, wherein said first antigen-binding region comprises a CDR1 sequence as set forth in SEQ ID NO:1, a CDR2 sequence as set forth in SEQ ID NO:2 and a CDR3 sequence as set forth in SEQ ID NO:3.

In another embodiment, the first antigen-binding region is humanized, wherein preferably the antigen-binding region comprises or consists of:
- the sequence set forth in SEQ ID NO:4, or
- a sequence having at least 90%, such as at least 92%, e.g. at least 94%, such as at least 96%, e.g. at least 98% sequence identity to the sequence set forth in SEQ ID NO:4.

In one embodiment, X₁ in SEQ ID NO:4 is S (Ser). In another embodiment, X₁ in SEQ ID NO:4 is G (Gly). In one embodiment, X₂ in SEQ ID NO:4 is F (Phe). In another embodiment, X₂ in SEQ ID NO:4 is S (Ser). In one embodiment, X₁ in SEQ ID NO:4 is S (Ser) and X₂ in SEQ ID NO:4 is F (Phe). In one embodiment, X₁ in SEQ ID NO:4 is S (Ser) and X₂ in SEQ ID NO:4 is S (Ser). In one embodiment, X₁ in SEQ ID NO:4 is G (Gly) and X₂ in SEQ ID NO:4 is F (Phe). In one embodiment, X₁ in SEQ ID NO:4 is G (Gly) and X₂ in SEQ ID NO:4 is S (Ser).

In some embodiments, the antibody in the pharmaceutical composition of the invention is a multispecific antibody, such as a bispecific antibody. Thus, in one embodiment, the antibody further comprises a second antigen-binding region. In one embodiment, the second antigen-binding region is a single-domain antibody.

In a further embodiment, the antibody is a bispecific antibody wherein both the first antigen-antigen binding region and the second antigen-binding region are single-domain antibodies. In a further embodiment, the multispecific antibody is a bispecific antibody, wherein the first antigen-binding region is a single-domain antibody and the second antigen-binding region is a single-domain antibody.

In one embodiment, the antibody comprised within the pharmaceutical composition of the invention comprises a second antigen-binding region and the second antigen-binding region is capable of binding human EGFR. Bispecific antibodies targeting both Vγ9Vδ2-T cells and EGFR have been shown to induce potent Vγ9Vδ2 T cell activation and tumor cell lysis both *in vitro* and in an *in vivo* mouse xenograft model (de Bruin et al. (2018) Oncoimmunology 1, e1375641).

In a further embodiment, the antibody comprises a second antigen-binding region and the second antigen-binding region comprises the CDR1 sequence set forth in SEQ ID NO:5, the CDR2 sequence set forth in SEQ ID NO:6 and the CDR3 sequence set forth in SEQ ID NO:7.

In one embodiment, the second antigen-binding region is humanized.

In a further embodiment, the antibody comprises a second antigen-binding region and the second antigen-binding region comprises or consists of
- the sequence set forth in SEQ ID NO:8, or
- a sequence having at least 90%, such as at least 92%, e.g. at least 94%, such as at least 96%, e.g. at least 98% sequence identity to the sequence set forth in SEQ ID NO:8.

In a further embodiment, the antibody competes (i.e. is able to compete) for binding to human EGFR with an antibody having the sequence set forth in SEQ ID NO:8, preferably the antibody binds the same epitope on human EGFR as an antibody having the sequence set forth in SEQ ID NO:8.

In a further embodiment, the antibody of the invention comprises a first antigen-binding region and a second antigen-binding region, wherein the first antigen-binding region comprises the CDR1 sequence set forth in SEQ ID NO:1, the CDR2 sequence set forth in SEQ ID NO:2 and the CDR3 sequence set forth in SEQ ID NO:3 and wherein the second antigen-binding region comprises the CDR1 sequence set forth in SEQ ID NO:5, the CDR2 sequence set forth in SEQ ID NO:6 and the CDR3 sequence set forth in SEQ ID NO:7.

In a further embodiment, the antibody of the invention comprises a first antigen-binding region and a second antigen-binding region, wherein the first antigen-binding region comprises the sequence set forth in SEQ ID NO:4 and the second antigen-binding region comprises the sequence set forth in SEQ ID NO:8. In further embodiments hereof:
X₁ in SEQ ID NO:4 is S (Ser) and X₂ in SEQ ID NO:4 is F (Phe), or
X₁ in SEQ ID NO:4 is S (Ser) and X₂ in SEQ ID NO:4 is S (Ser), or
X₁ in SEQ ID NO:4 is G (Gly) and X₂ in SEQ ID NO:4 is F (Phe), or
X₁ in SEQ ID NO:4 is G (Gly) and X₂ in SEQ ID NO:4 is S (Ser).

In a further embodiment, the antibody is capable of mediating killing of human EGFR-expressing cells. In a preferred embodiment, the antibody is able to increase Vγ9Vδ2 T cell mediated killing of EGFR expressing cells, such as A431 cell at least 25%, such as at least 50%, e.g. at least 2-fold, when tested as described in

### Example 9 herein.

In a further embodiment, the antibody is not capable of mediating killing of EGFR-negative cells, such as EGFR negative human cells.

In one embodiment, the antibody comprises a first antigen-binding region and a second antigen-binding region wherein the first antigen-binding region and second antigen-binding region are covalently linked via a peptide linker, e.g. a linker having a length of from 1 to 20 amino acids, e.g. from 1 to 10 amino acids, such as 2, 3, 4, 5, 6, 7, 8 or 10 amino acids. In one embodiment, the peptide linker comprises or consists of the sequence GGGGS, set forth in SEQ ID NO:9.

In another embodiment, the antibody comprises a first antigen-binding region and a second antigen-binding region, wherein the first antigen-binding region capable of binding human Vδ2 is located C-terminally of the second antigen-binding region capable of binding a human EGFR.

In one embodiment, the antibody comprised within the pharmaceutical composition of the invention further comprises a half-life extension domain. In one embodiment, the antibody has a terminal half-life that is longer than about 168 hours when administered to a human subject. Most preferably the terminal half-life is 336 hours or longer. The "terminal half-life" of an antibody, when used herein refers to the time taken for the serum concentration of the polypeptide to be reduced by 50%, in vivo in the final phase of elimination.

In one embodiment, the antibody further comprises a half-life extension domain and the half-life extension domain is an Fc region. In a further embodiment, the antibody is a multispecific antibody, such as a bispecific antibody comprising an Fc region. Various methods for making bispecific antibodies have been described in the art, e.g. reviewed by Brinkmann and Kontermann (2017) MAbs 9:182. In one embodiment of the present invention, the Fc region is a heterodimer comprising two Fc polypeptides, wherein the first antigen-binding region is fused to the first Fc polypeptide and the second antigen-binding region is fused to the second Fc polypeptide and wherein the first and second Fc polypeptides comprise asymmetric amino acid mutations that favor the formation of heterodimers over the formation of homodimers. (see e.g. Ridgway et al. (1996) 'Knobs-into-holes' engineering of antibody CH3 domains for heavy chain heterodimerization. Protein Eng 9:617). In a further embodiment hereof, the CH3 regions of the Fc polypeptides comprise said asymmetric amino acid mutations, preferably the first Fc polypeptide comprises a T366W substitution and the second Fc polypeptide comprises T366S, L368A and Y407V substitutions, or vice versa, wherein the amino acid positions correspond to human IgG1 according to the EU numbering system. In a further embodiment, the cysteine residues at position 220 in the first and second Fc polypeptides have been deleted or substituted, wherein the amino acid position corresponds to human IgG1 according to the EU numbering system. In a further embodiment, the region comprises the hinge sequence set forth in SEQ ID NO:10.

In some embodiments, the first and/or second Fc polypeptides contain mutations that render the antibody inert, i.e. unable to, or having reduced ability to, mediate effector functions. In one embodiment, the inert Fc region is in addition not able to bind C1q. In one embodiment, the first and second Fc polypeptides comprise a mutation at position 234 and/or 235, preferably the first and second Fc polypeptide comprise an L234F and an L235E substitution, wherein the amino acid positions correspond to human IgG1 according to the EU numbering system. In another embodiment, the antibody contains a L234A mutation, a L235A mutation and a P329G mutation. In another embodiment, the antibody contains a L234F mutation, a L235E mutation and a D265A mutation.

In a preferred embodiment, the first antigen-binding region comprises the sequence set forth in SEQ ID NO:4, the second antigen-binding region comprises the sequence set forth in SEQ ID NO:8 and
- the first Fc polypeptide comprises the sequence set forth in SEQ ID NO:11 and the second Fc polypeptide comprises the sequence set forth in SEQ ID NO:12, or
- the first Fc polypeptide comprises the sequence set forth in SEQ ID NO:12 and
   the second Fc polypeptide comprises the sequence set forth in SEQ ID NO:11. In one embodiment, X₃ in SEQ ID NO:11 is K. In another embodiment, X₃ in SEQ ID NO:11 is absent.

In one embodiment, X₃ in SEQ ID NO:12 is K. In another embodiment, X₃ in SEQ ID NO:12 is absent.

In a further preferred embodiment, the antibody comprises or consists of the sequences set forth in SEQ ID NO:16 and SEQ ID NO:17.

In one embodiment, X₃ in SEQ ID NO:16 is K. In another embodiment, X₃ in SEQ ID NO:16 is absent.

In one embodiment, X₃ in SEQ ID NO:17 is K. In another embodiment, X₃ in SEQ ID NO:17 is absent.

In a further preferred embodiment, the antibody comprises or consists of the sequences set forth in SEQ ID NO:16 and SEQ ID NO:18.

In one embodiment, X₃ in SEQ ID NO:18 is K. In another embodiment, X₃ in SEQ ID NO:18 is absent.

In a further main aspect, the invention relates to the pharmaceutical composition according to the invention as described herein for use as a medicament.

Antibodies used in the pharmaceutical compositions according to the invention enables creating a microenvironment that is beneficial for killing of tumor cells by Vγ9Vδ2 T cells. Accordingly, in a preferred embodiment, the antibody is for use in the treatment of cancer.

In one embodiment, the pharmaceutical composition is for use in the treatment of primary or metastatic colon or colorectal cancer. In another embodiment, the pharmaceutical composition is for use in the treatment of cancer of the peritoneum. In another embodiment, the pharmaceutical composition is for use in the treatment of liver cancer. In another embodiment, the pharmaceutical composition is for use in the treatment of head and neck squamous cell carcinoma (HNSCC). In another embodiment, the pharmaceutical composition is for use in the treatment of non-small cell lung carcinoma (NSCLC). In another embodiment, the pharmaceutical composition is for use in the treatment of squamous cell carcinoma of the skin.

Similarly, the invention relates to a method of treating a disease comprising administration of a pharmaceutical composition according to the invention as described herein to a human subject in need thereof. In one embodiment, the disease is cancer.

In some embodiments, the pharmaceutical composition is administered as monotherapy. However, pharmaceutical compositions of the present invention may also be administered in combination therapy, i.e., combined with other therapeutic agents relevant for the disease or condition to be treated.

"Treatment" or "treating" refers to the administration of an effective amount of a pharmaceutical composition according to the present invention with the purpose of easing, ameliorating, arresting, eradicating (curing) or preventing symptoms or disease states. An "effective amount" refers to an amount effective, at dosages and for periods of time necessary, to achieve a desired therapeutic result. An effective amount of a polypeptide, such as an antibody, may vary according to factors such as the disease stage, age, sex, and weight of the individual, and the ability of the antibody to elicit a desired response in the individual. An effective amount is also one in which any toxic or detrimental effects of the antibody are outweighed by the therapeutically beneficial effects. Administration may be carried out by any suitable route, but will typically be parenteral, such as intravenous, intramuscular or subcutaneous.

Multispecific antibodies used in the invention are typically produced recombinantly, i.e. by expression of nucleic acid constructs encoding the antibodies in suitable host cells, followed by purification of the produced recombinant antibody from the cell culture. Nucleic acid constructs can be produced by standard molecular biological techniques well-known in the art. The constructs are typically introduced into the host cell using an expression vector. Suitable nucleic acid constructs and expression vectors are known in the art. Host cells suitable for the recombinant expression of antibodies are well-known in the art, and include CHO, HEK-293, Expi293F, PER-C6, NS/0 and Sp2/0 cells.

**Table 1: Sequence listing.**

| SEQ ID. | code | Descripti on | Sequence |
|---|---|---|---|
| 1 | 5C8 var | CDR1 | NYAMX₁, wherein X₁ is S or G |
| 2 | 5C8 var | CDR2 | AISWSGGSTSYADSVKG |
| 3 | 5C8 var | CDR3 | QFSGADX₂GFGRLGIRGYEYDY, wherein X₂ is F or S |
| 4 | 5C8 var | VHH | EVQLLESGGGSVQPGGSLRLSCAASGRPFSNYAM X1WFRQAPGKEREFVSAISWSGGSTSYADSVKGRFTIS RDNSKNTLYLQMNSLRAEDTAVYYCAAQFSGAD X2GFGRLGIRGYEYDYWGQGTQVTVSS, wherein X1 is S or G, and wherein X2 is F or S |
| 5 | 7D12 | CDR1 | SYGMG |
| 6 | 7D12 | CDR2 | GISWRGDSTGYADSVKG |
| 7 | 7D12 | CDR3 | AAGSAWYGTLYEYDY |
| 8 | 7D12var 8 | VHH | EVQLVESGGGSVQPGGSLRLSCAASGRTSRSYGMGW FRQAPGKEREFVSGISWRGDSTGYADSVKGRFTISRD NAKNTVDLQMNSLRAEDTAVYYCAAAAGSAWYGTLYE YDYWGQGTLVTVSS |
| 9 | linker | | GGGGS |
| 10 | | Modified hinge | AAASDKTHTCPPCP |
| 11 | IgG1 | Heavy chain constant region variant | AAASDKTHTCPPCPAPEFEGGPSVFLFPPKPKDTLMISR TPEVTCVVVDVSHEDPEVKFNWYVDGVEVHNAKTKPR EEQYNSTYRVVSVLTVLHQDWLNGKEYKCKVSNKALP APIEKTISKAKGQPREPQVYTLPPSRDELTKNQVSLWCL VKGFYPSDIAVEWESNGQPENNYKTTPPVLDSDGSFFL |
| | L234F | | |
| | L235E | | |
| | T366W | | |
| | | | YSKLTVDKSRWQQGNVFSCSVMHEALHNHYTQKSLSL SPGX3, wherein X3 is K or is absent |
| 12 | IgG1 | Heavy chain constant region variant | AAASDKTHTCPPCPAPEFEGGPSVFLFPPKPKDTLMISR TPEVTCVVVDVSHEDPEVKFNWYVDGVEVHNAKTKPR EEQYNSTYRVVSVLTVLHQDWLNGKEYKCKVSNKALP APIEKTISKAKGQPREPQVYTLPPSRDELTKNQVSLSCA VKGFYPSDIAVEWESNGQPENNYKTTPPVLDSDGSFFL VSKLTVDKSRWQQGNVFSCSVMHEALHNHYTQKSLS LSPGX3, wherein X3 is K or is absent |
| | L234F | | |
| | L235E | | |
| | T366S | | |
| | L368A | | |
| | Y407V | | |
| 13 | 5C8 | VHH | EVQLVESGGGLVQAGGSLRLSCAASGRPFSNYAMGWF RQAPGKEREFVAAISWSGGSTSYADSVKGRFTISRDN AKNTVYLQMNSPKPEDTAIYYCAAQFSGADYGFGRLGI RGYEYDYWGQGTQVTVSS |
| 14 | 5C8var1 | VHH | EVQLLESGGGSVQPGGSLRLSCAASGRPFSNYAMSWF RQAPGKEREFVSAISWSGGSTSYADSVKGRFTISRDN SKNTLYLQMNSLRAEDTAVYYCAAQFSGADYGFGRLGI RGYEYDYWGQGTQVTVSS |
| 15 | 5C8 | CDR3 | QFSGADYGFGRLGIRGYEYDY |
| 16 | 7D12var 8-Fc | VHH-Fc | EVQLVESGGGSVQPGGSLRLSCAASGRTSRSYGMGW FRQAPGKEREFVSGISWRGDSTGYADSVKGRFTISRD NAKNTVDLQMNSLRAEDTAVYYCAAAAGSAWYGTLYE YDYWGQGTLVTVSSAAASDKTHTCPPCPAPEFEGGPS VFLFPPKPKDTLMISRTPEVTCVVVDVSHEDPEVKFNW YVDGVEVHNAKTKPREEQYNSTYRVVSVLTVLHQDWL NGKEYKCKVSNKALPAPIEKTISKAKGQPREPQVYTLPP SRDELTKNQVSLSCAVKGFYPSDIAVEWESNGQPENN YKTTPPVLDSDGSFFLVSKLTVDKSRWQQGNVFSCSV MHEALHNHYTQKSLSLSPGX₃, wherein X₃ is K or is absent |
| 17 | 5C8var1 (Y105F)-Fc | VHH-Fc | EVQLLESGGGSVQPGGSLRLSCAASGRPFSNYAMSWF RQAPGKEREFVSAISWSGGSTSYADSVKGRFTISRDN SKNTLYLQMNSLRAEDTAVYYCAAQFSGADFGFGRLGI RGYEYDYWGQGTQVTVSSAAASDKTHTCPPCPAPEFE GGPSVFLFPPKPKDTLMISRTPEVTCVVVDVSHEDPEV KFNWYVDGVEVHNAKTKPREEQYNSTYRVVSVLTVLH QDWLNGKEYKCKVSNKALPAPIEKTISKAKGQPREPQV YTLPPSRDELTKNQVSLWCLVKGFYPSDIAVEWESNG QPENNYKTTPPVLDSDGSFFLYSKLTVDKSRWQQGNV FSCSVMHEALHNHYTQKSLSLSPGX3, wherein X3 is K or is absent |
| 18 | 5C8var1 (Y105S)-Fc | VHH-Fc | EVQLLESGGGSVQPGGSLRLSCAASGRPFSNYAMSWF RQAPGKEREFVSAISWSGGSTSYADSVKGRFTISRDN SKNTLYLQMNSLRAEDTAVYYCAAQFSGADSGFGRLGI RGYEYDYWGQGTQVTVSSAAASDKTHTCPPCPAPEFE GGPSVFLFPPKPKDTLMISRTPEVTCVVVDVSHEDPEV KFNWYVDGVEVHNAKTKPREEQYNSTYRVVSVLTVLH QDWLNGKEYKCKVSNKALPAPIEKTISKAKGQPREPQV YTLPPSRDELTKNQVSLWCLVKGFYPSDIAVEWESNGQ PENNYKTTPPVLDSDGSFFLYSKLTVDKSRWQQGNVFS CSVMHEALHNHYTQKSLSLSPGX3, wherein X3 is K or is absent |

All references, articles, publications, patents, patent publications, and patent applications cited herein are incorporated by reference in their entireties for all purposes. However, mention of any reference, article, publication, patent, patent publication, and patent application herein is not, and should not be, taken as acknowledgment or any form of suggestion that they constitute valid prior art or form part of the common general knowledge in any country in the world.

### EXAMPLES

### Example 1| Production and purification of VHH compounds

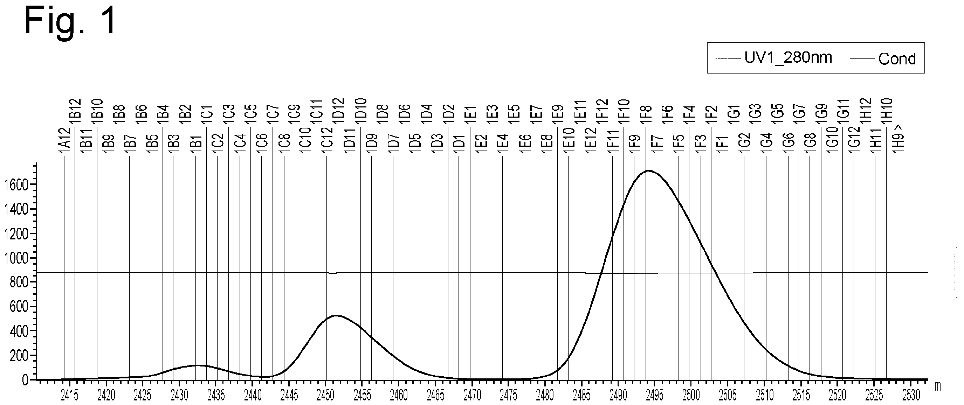

VHH compounds were mostly produced by transient transfection of the encoding plasmids in HEK293-E 253 cells and purification of the proteins from the conditioned medium (after a week of production) by protein-A affinity chromatography, followed by preparative gel filtration. The dominant monomeric peak (fractions 1E11-1G2) observed in preparative size exclusion using a Superdex-75 column was purified: Figure 1.

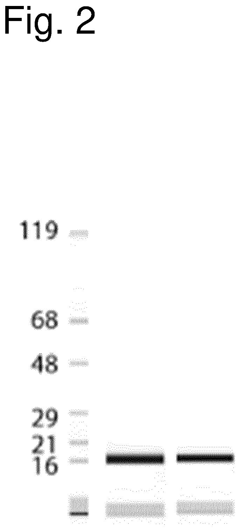

Purified proteins were shown to migrate as a single band under reducing- and non-reducing conditions in polyacrylamide gel electrophoresis: Figure 2 shows a representative example.

### Example 2| HP-SEC analysis of purified VHH compounds

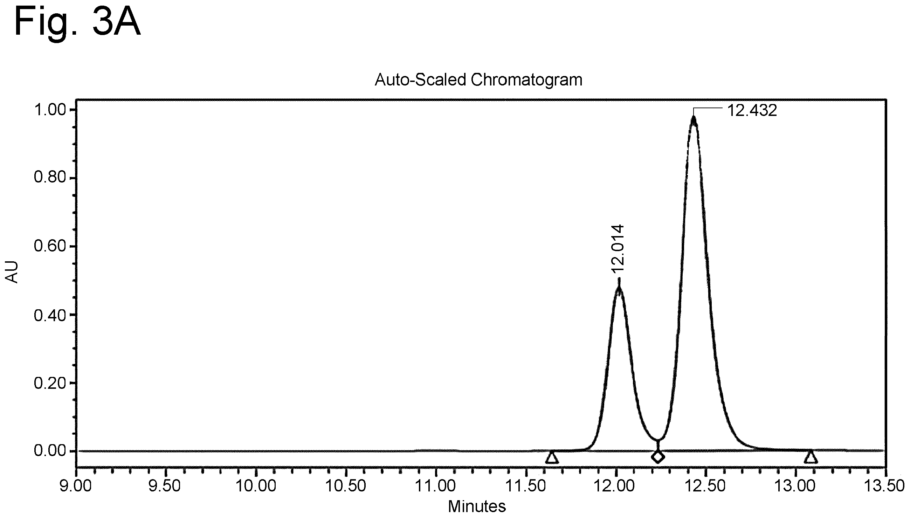

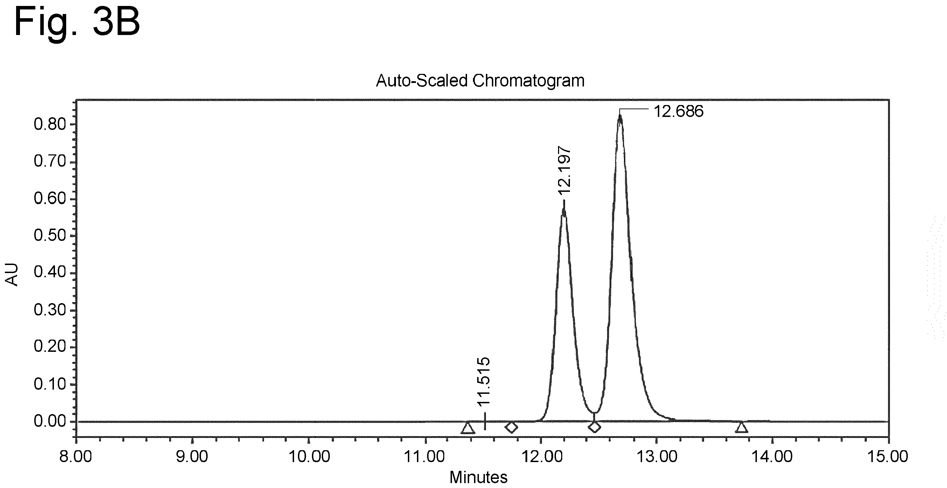

The Waters Acquity ARC-bio system was used for HP-SEC analysis of purified VHH compounds. 10µg of antibody (10µL of antibody with a concentration of 1mg/mL) was injected on a Waters BEH200 SEC column (bead size 2.5µm, column dimensions 7.8 x 300 mm). The mobile phase consisted of 50mM Sodium Phosphate, 0.2M Sodium Chloride buffer pH7.0 and a buffer velocity of 0.8mL/min was used for the run. Protein was detected by measuring absorption at a wavelength of 214nm. The total analysis time was 15 minutes per injection. VHH compounds were produced and purified as described in Example 1. Surprisingly, when VHH 5C8 (SEQ ID NO:13) (previously described in WO2015156673) and 5C8var1 (SEQ ID NO:14) (previously described in WO2020060405) were tested in HP-SEC analysis for integrity and monomericity, two peaks were observed: Figure 3.

### Example 3| Mass spec analysis of 5C8 reveals an additional mass of 80Da

To determine whether the two isoforms of 5C8 differed in mass, the protein preparation of 5C8 was analysed by LC-ESI-MS Mass spectrometry. The main species found in this analysis was 5C8 without post translational modifications or signal peptide. A second species was a protein with a mass difference of + 80.3 Dalton (Da), which indicated possible sulfation or phosphorylation. This was further investigated by treatment with phosphatase or sulfatase and subsequent LC-ESI-MS mass spec analysis of peptides after proteolytic digestion. It was shown that sulfatase treatment reduced the mass of the peptides containing Y105, the 7^{th} residue of the CDR3 (SEQ ID NO:15) by 80Da, whereas phosphatase treatment had no effect. This proves the Y105 in 5C8 to be post-translationally modified by sulfation. This sulfation was present in roughly 30% of the protein preparation.

### Example 4| 1D12var5-5C8var1 containing the same anti-Vγ9Vδ2 VHH shows the same heterogeneity in HP-SEC and the same extra mass of 80Da

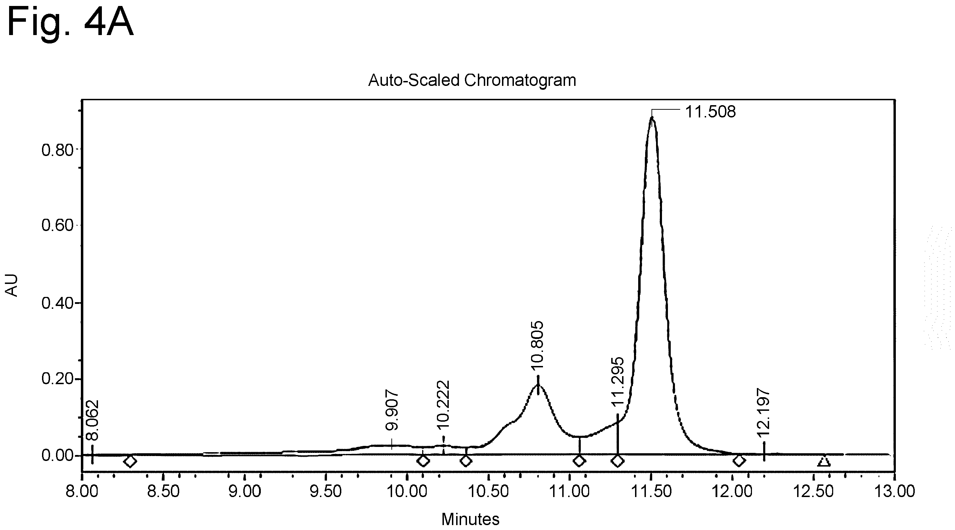

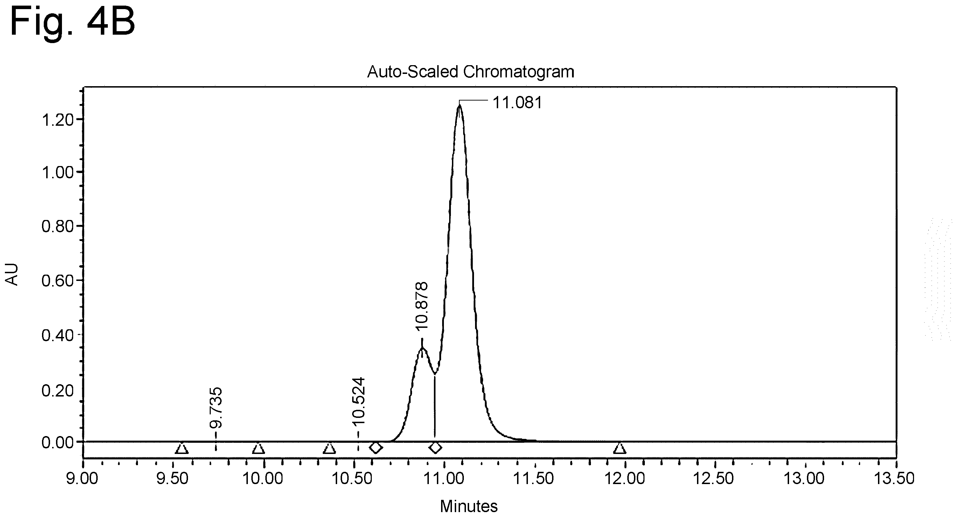

1D12var5-5C8var1 is a bispecific VHH compound composed of an anti-CD1d VHH, coupled via a flexible linker to 5C8var1 (described in SEQ ID NO:87 in WO2020060405). This protein was expressed in HEK 293 E cells as described above. In addition, protein preparations were obtained from different expression systems: the bispecific VHH was also expressed in *Pichia pastoris* and in Chinese hamster ovarian (CHO) cells. When different protein preparations were tested in HP-SEC analysis, a pre-peak was systematically observed: Figure 4.

The pre-peak observed is indicative of a significant percentage of the protein being a different isoform again. As the 5C8 VHH was shown to be sulphated and the 5C8var1 contains the exact same CDR3 sequence, the 1D12var5-5C8var1 batches were also analysed by mass spectrometry for their molecular weight. Dependent on the protein batch, between 15% and 40% was found to contain an additional mass of 80Da. This is consistent with a sulfation, as observed for VHH 5C8.

### Example 5| In silico analysis of VHH 5C8 and 5C8var1

A homology model of 5C8 and 5C8var1 was built using Maestro (Schrödinger) based on PDB ID 5M2W. CDR1 and CDR3 required refinement by de novo loop prediction using Prime (Schrödinger). The generated models demonstrated that CDR3 residue Y105 shows a high solvent-accessibility surface area of 205.1 Å² in the model of 5C8var1 and 122.2 Å² in the model of 5C8 and is therefore expected to contribute to antigen binding. Subsequently, the models were analyzed for reactive residues, indicating residues that are prone to post-translational modifications (PTM). Next, the protein sequences were analyzed using ModPred, a sequence based PTM prediction tool. Modifications that were predicted in both structure and sequence are listed in table 2. The individual predicted PTMs could not explain the mass difference observed in HP-SEC analyses.

**Table 2| Predicted PTMs in Maestro and ModPred for 5C8 and 5C8var1. Type describes the PTM as predicted by Maestro. ^{∗}Q13 deamidation was only predicted for 5C8.**

| **Residue number** | **Residue** | **Type** |
|---|---|---|
| 13^{∗} | Q | Deamidation |
| 32 | Y | Oxidation |
| 39 | Q | Deamidation |
| 60 | Y | Oxidation |
| 62 | D | Proteolysis (ASP) |
| 73 | D | Proteolysis (ASP) |
| 74 | N | Deamidation |
| 80 | Y | Oxidation |
| 84 | N | Deamidation |
| 90 | D | Proteolysis (ASP) |
| 94 | Y | Oxidation |
| 95 | Y | Oxidation |
| 104 | D | Proteolysis (ASP) |
| 105 | Y | Oxidation |
| 115 | Y | Oxidation |
| 117 | Y | Oxidation |
| 118 | D | Proteolysis (ASP) |
| 119 | Y | Oxidation |
| 122 | Q | Deamidation |

### Example 6| Design and production of 5C8var1 VHH CDR3 mutants Y105F and Y105S

The homology models, as described in Example 5, were used to introduce mutations that would prevent sulfation of Y105 in 5C8 and 5C8var1. Two different mutants were designed on the basis of a model structure of the VHH: Y105S (retaining the alcohol function) and Y105F (retaining the aromatic ring). Residue Y105 is the 7^{th} residue of CDR3; by introducing mutations, an effect on binding was expected. Both mutations were designed in the humanized VHH sequence 5C8var1 and both proteins were produced in HEK293E cells and purified as described above. The CDR3 amino acid sequence of the humanized VHH was kept identical to the one of the non-humanised.

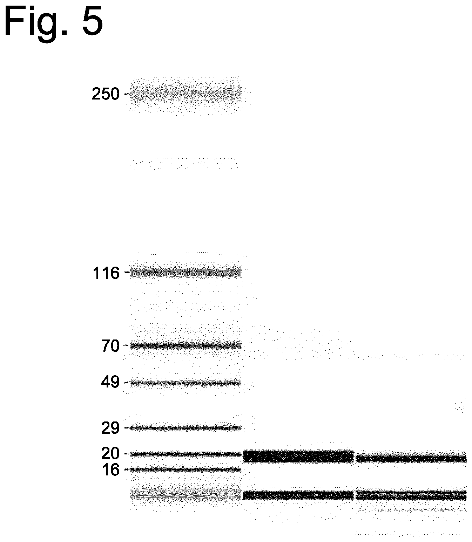

Both 5C8var1-Y105F and 5C8var1-Y105S were well produced and appeared as monomeric proteins in preparative size exclusion (data not shown). Both proteins were highly pure (Figure 5) and migrated as a single species in polyacrylamide gel electrophoresis.

### Example 7| HP-SEC analysis of purified VHH containing designed CDR3 mutations

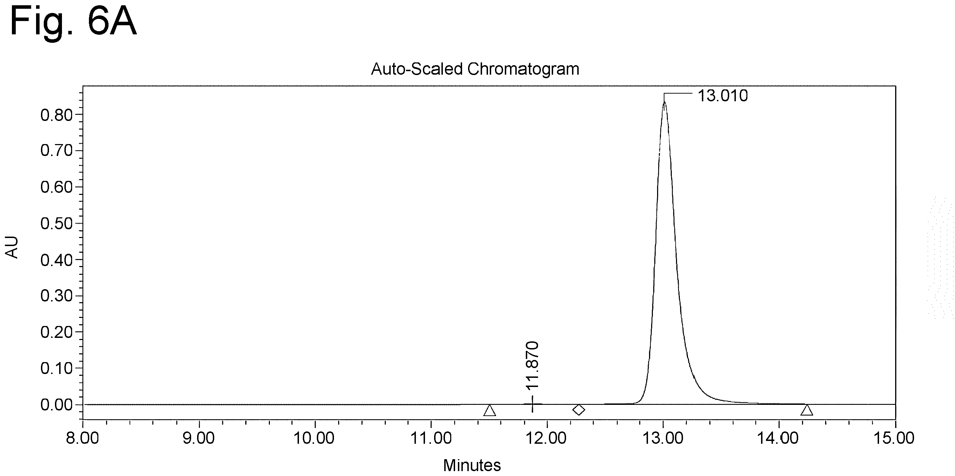

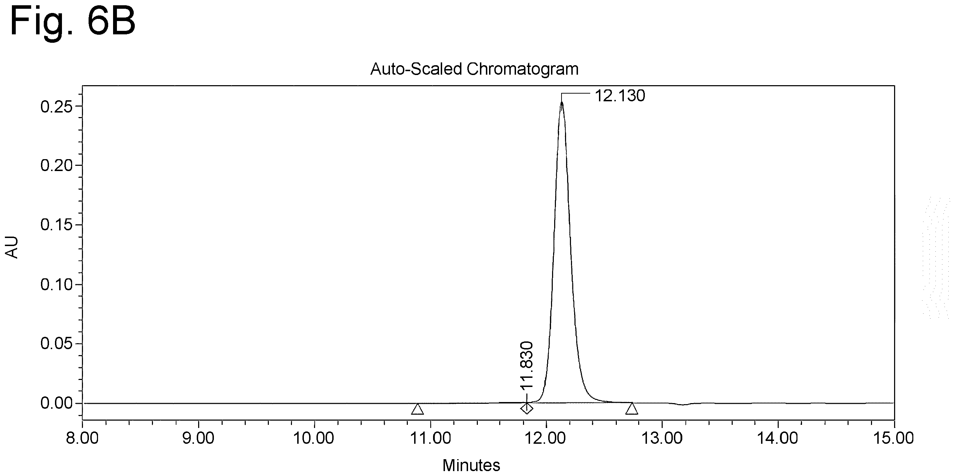

HP-SEC analysis was performed as described for 5C8. Both 5C8var1-Y105F as well as 5C8var1-Y105S were analysed (Figure 6).

As can be concluded from the HP-SEC analysis of the purified VHH molecules containing the designed CDR3 mutations, no heterogeneity was observed anymore for either mutant. This indicated that the observed Y105 post translational modification was absent and the proteins were homogeneous.

### Example 8| Affinity measurement of 5C8var1, 5C8var1-Y105F and 5C8va1-Y105S using Biolayer Interferometry (BLI) shows no difference in affinity

Binding of the 5C8var1 VHH antibody fragment and variants 5C8var1-Y105F and 5C8var1-Y105S to the Vy9Vδ2TCR was measured by biolayer interferometry using an Octet RED96 instrument (ForteBio). Recombinant human Vy9Vδ2-Fc fusion protein (20 µg/ml) was captured as ligand on anti-human Fc capture biosensors. Sensorgrams were recorded when ligand captured biosensors were incubated with a dilution series of VHH antibody fragments (40 to 0.63 nM) in 10X kinetics buffer (ForteBio). Global data fitting to a 1:1 binding model was used to estimate values for the kₒₙ (association rate constant) and _{koff} (dissociation rate constant). These values were used to calculate the KD (equilibrium dissociation constant) using KD = k_{off}/kₒₙ.

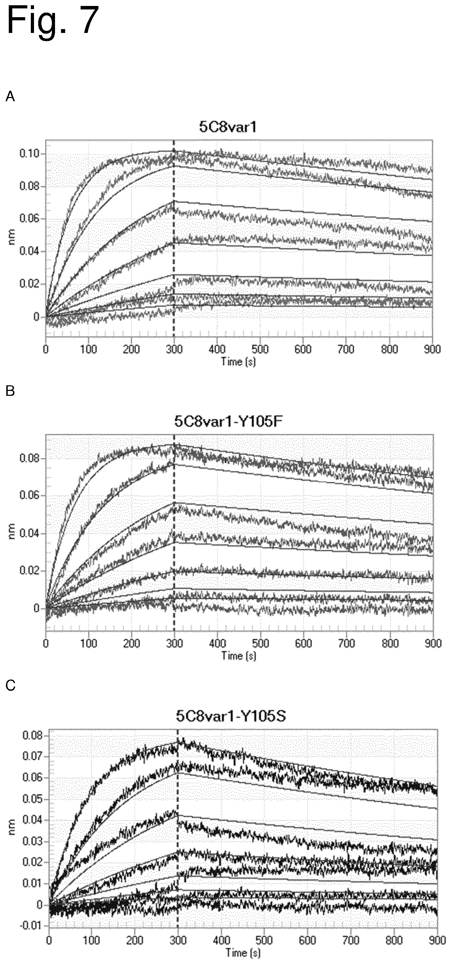

As can be concluded from figure 7 and from Table 3, the KD values found for the two different Y105 VHH mutants did not differ substantially from the value found for 5C8var1. Especially the Y105F mutant had a comparable affinity to that found for 5C8var1.

**Table 3| Affinity values found in BLI measurements of VHH binding to recombinant Vγ9Vδ2 TCR protein. The values depicted are the means of at least three independent measurements +/- standard deviation.**

| **VHH compound** | **KD (nM) +/- SD** |
|---|---|
| 5C8var1 | 0.81 +/- 0.02 |
| 5C8var1-Y105F | 0.78 +/- 0.23 |
| 5C8var1-Y105S | 1.59 +/- 0.31 |

### Example 9| The functionality of an anti-(EGFR x Vγ9Vδ2 TCR) bispecific VHH containing the Y105F mutation is fully retained

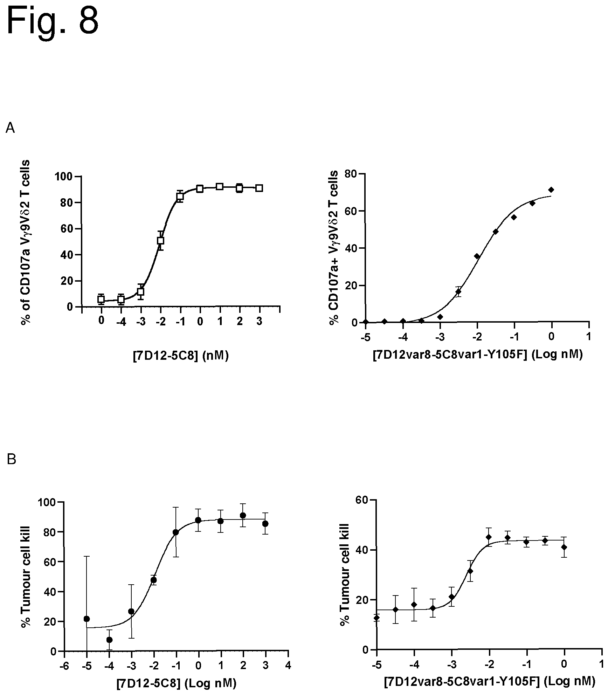

To determine whether the equal affinity of the VHH 5C8var1-Y105F compared to that of 5C8var1 could be translated into a comparable functionality, the bispecific VHH 7D12var8-5C8var1-Y105F was designed: a humanized anti-EGFR VHH 7D12var8 (based on the VHH described in Gainkam et al. (2008) J Nucl Med 49(5):788) was coupled via a G4S linker to the 5C8var1-Y105F VHH, forming 7D12var8-5C8var1-Y105F. The two VHH molecules were separated by a flexible G4S linker sequence. This molecule was produced and purified as described above and then tested for its ability to induce Vγ9Vδ2 T cell activation dependent on an EGFR positive tumor cell line (A431) and to cause T cell-mediated tumor cell lysis. Briefly, Vγ9Vδ2 T cells were isolated from the blood of healthy donors using magnetic activated cell sorting (MACS) in combination with an anti-Vδ2 antibody according to standardized procedures. These cells were then expanded for a week using a mix of cytokines and irradiated feeder cells: a mix of the JY cell line and PBMC from a different donor. Vγ9Vδ2 T cells were always >90% pure (stained double positive for Vy9 and for Vδ2 in FACS) when used in an assay. The A431 cell line (ATCC, cat nr. CRL-1555) was cultured according to the supplier's recommendation. For an activation or cytotoxicity assay, 50,000 tumor target cells were plated in 96 wells tissue culture plates the day before the assay. The next day, 50,000 expanded, purified Vγ9Vδ2 T cells were added in medium together with a concentration range of bispecific VHH compound. In the activation assay, Vy9Vδ2-T cell degranulation was assessed using a mix of a labeled anti-CD3 and anti-CD107A antibodies that was added to the mix. After 4 hours, cells were harvested, washed and analysed by FACS for expression of the degranulation marker CD107A. For the cytotoxicity assay, the supernatants of the co-cultures were examined the day after for the presence of protease (indicative of cell death) using the CytoTox-Glo cytotoxicity assay kit: (Promega G9290). Cell lysis using detergent was used to set 100% killing at the end of the assay. Figure 8 shows the data.

Figure 8 shows that 7D12var8-5C8var1-Y105F, as well as the non-humanised 7D12-5C8 induced potent Vγ9Vδ2 T cell activation and tumor cell lysis. These results are in line with the potency of the non-humanised 'precursor' molecule without the Y105 mutation 7D12wt-5C8. Table 4 shows the EC50 values that were obtained after curve fitting. 7D12var8-5C8var1-Y105F had a slightly lower EC50 in the cytotoxicity assay as compared to 7D12-5C8.

**Table 4| EC50 values found after curve fitting of the data represented in Figure 8 using GraphPad software.**

| **Antibody used** | **EC50 degranulation (pM)** | **EC50 cytotoxicity (pM)** |
|---|---|---|
| 7D12-5C8 | 10 | 9 |
| 7D12var8-5C8var1-Y105F | 11 | 2.5 |

The maximal level of tumor cell kill was slightly lower in case of 7D12var8-5C8var1-Y105F, compared to the level of tumor cell kill observed for 7D12-5C8. However, these were two different measurements using two different Vγ9Vδ2 T cell donors and this maximal level of cytotoxicity may be particularly donor-dependent.

### Example 101 The temperature stability of VHH 5C8var1 containing the Y105 mutations was not changed

To determine whether the mutation introduced in the different variants affected the thermostability of the VHH folding, the melting temperatures of the mutants were measured using NanoDSF (Differential Scanning Fluorimetry). Antibody samples were diluted using PBS until they were equal to the sample with the lowest concentration. The antibody samples were subsequently filled in nanoDSF grade capillaries and measured with the Prometheus NT.48. During the experiment, temperature was ramped from 20 to 95°C. The intrinsic fluorescence of the protein was detected at 350 and 330 nm and was recorded together with the amount of reflected light. From these measurements, the apparent melting temperature (Tm) and aggregation onset (Tagg) were determined. For all three antibody fragments, the onset melting temperature (Tₒₙ) and melting temperature (Tm) at which the VHH were completely unfolded were reported (Table 5). Melting temperatures measured for 5C8var1-Y105F and 5C8var1-Y105S were in line with that of 5C8var1: Table 5.

**Table 5| 5C8var1-Y105F and 5C8var1-Y105S show similar melting temperatures as 5C8var1, indicating stability is not impaired by the introduced mutations.**

| | Tₒₙ (°C) | Tm (°C) |
|---|---|---|
| 5C8var1 | 61.8 | 86.7 |
| 5C8var1-Y105F | 64.5 | 85.5 |
| 5C8var1-Y105S | 63.9 | 86.6 |

### Example 11| Half-life extended (Fc containing) bispecific constructs

In order to obtain a molecule with a longer *in vivo* plasma half-life, the 7D12var8-5C8var1-Y105F bispecific VHH was re-formatted into a therapeutic antibody format containing a human Fc. Both VHH domains were coupled to a human IgG1 Fc (i.e. CH2 and CH3) domain with the following characteristics: the VHH was coupled to a modified hinge (AAA, followed by SDKTHTCPPCP, cysteine 220 was deleted) and human CH2 and CH3 domains. The CH2 domain was Fc-silenced by the LFLE mutational pair (L234F, L235E) and the CH3 domains were mutated with the 'knobs-into-holes' mutations (knob: T366W and hole: T366S, L368A and Y407V) that enforce hetero-dimerization, upon co-expression of the two chains in the same cell. This mutational pair has been described in the scientific literature (Ridgway et al. (1996) Protein Eng 9:617). The sequences of the construct are set forth in SEQ ID NO:16 and SEQ ID NO:17. The resulting antibody construct 7D12var8-5C8var1(Y105F) with Fc region was termed 7D12var8-5C8var1(Y105F)-Fc. Similarly, a construct was prepared wherein Y at position 105 was replaced with S (7D12var8-5C8var1(Y105S)-Fc). The sequences of that construct are set forth in SEQ ID NO:16 and SEQ ID NO:18.

Protein was made via co-transfection of the encoding two expression vectors in HEK293E cells and purification from the culture supernatant by means of protein-A affinity chromatography, followed by preparative size exclusion chromatography, as described in Example 1. This yielded a highly monomeric protein preparation.

### Example 12| Binding of 7D12var8-5C8var1(Y105F)-Fc to primary Vγ9Vδ2 T cells isolated from healthy human PBMCs

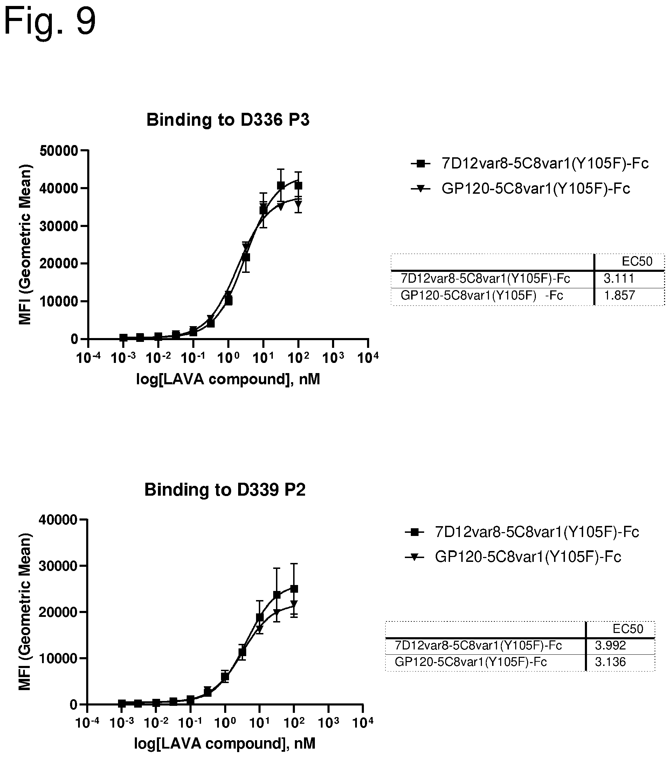

To demonstrate the binding of 7D12var8-5C8var1(Y105F)-Fc to the Vγ9Vδ2 T cell receptor (TCR), human Vγ9Vδ2 T cells were isolated from healthy peripheral blood mononuclear cells (PBMCs) by magnetic activated cell sorting (MACS) and subsequently expanded as described (de Bruin et al., Clin. Immunology 169 (2016), 128-138; de Bruin et al., J. Immunology 198(1) (2017), 308-317). Expanded polyclonal and pure (>95%) Vγ9Vδ2T cells were then seeded at a concentration of 50000 cells/well and incubated with either the 7D12var8-5C8var1(Y105F)-Fc antibodies or GP120-5C8var1(Y105F)-Fc antibodies as a positive control in a half-log titration starting at 100 nM for one hour at 4°C. Binding of the antibodies to the Vγ9Vδ2 TCR was visualized by flow cytometry using a fluorescently labelled secondary anti-IgGl antibody. Figure 9 shows the mean fluorescent intensity (MFI) signal of the anti-IgGl antibody staining as measured by flow cytometry for two different PBMC donors (D336 and D339). The sigmoidal curves underline a significant binding of the 7D12var8-5C8var1(Y105F)-Fc to Vγ9Vδ2 T cells with a half maximal effective concentration (EC50) in the low nanomolar range (~3 nM).

### Example 13| Binding of 7D12var8-5C8var1(Y105F)-Fc to EGFR positive tumor cells by cell-based ELISA

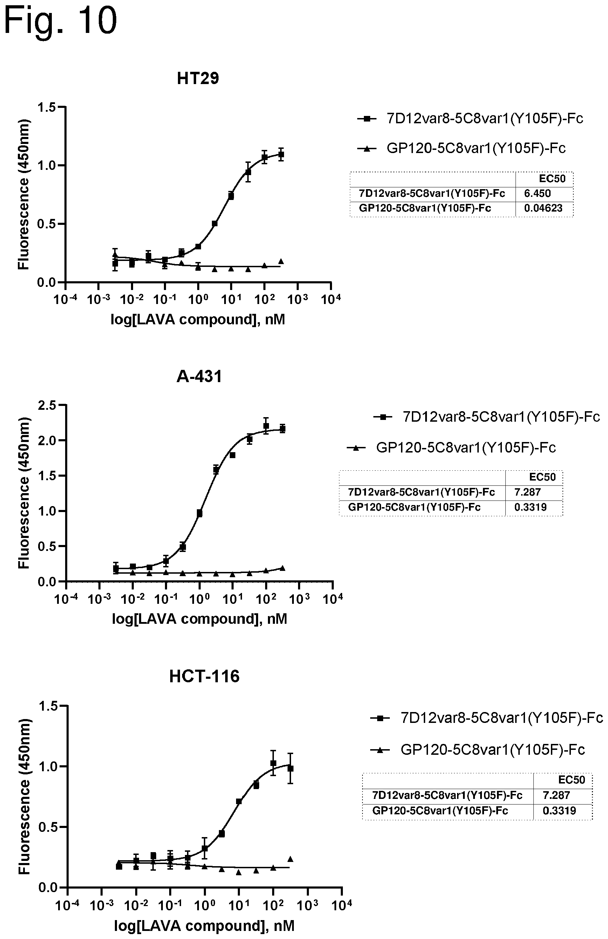

The binding of 7D12var8-5C8var1(Y105F)-Fc to the epidermal growth factor receptor (EGFR) was tested in a cell-based enzyme-linked immunosorbent assay (ELISA) using EGFR-expressing tumor cell lines A-431, HCT-116 and HT-29. To this end, tumor cells were first seeded at different concentrations on day -1 to reach a concentration of approximately 50000 cells/well on day 0. On day 0, a half-log titration of 7D12var8-5C8var1(Y105F)-Fc antibodies or GP120-5C8var1(Y105F)-Fc antibodies as a negative control was added to the tumor cells starting at 100 nM for one hour at 37°C. Bound antibodies were then labelled in a secondary incubation step with anti-IgGl-HRP for one hour at 37°C. The secondary antibody binding was then resolved by the addition of 3,3',5,5'-Tetramethylbenzidine and the colorimetric change induced by the HRP, followed by the addition of H2SO4 to stop the reaction. The optical density (OD) was then measured in a UV spectrometer at a wavelength of 450 nm. Figure 10 shows that 7D12var8-5C8var1(Y105F)-Fc strongly binds to A-431, HCT-116 and HT-29 tumor cells with an EC50 of ~7 nM, whereas the non-targeting control antibody did not measurably bind any of the cell lines tested.

### Example 14| Degranulation of Vγ9Vδ2 T cells dependent on A-431 cells induced by 7D12var8-5C8var1(Y105F)-Fc

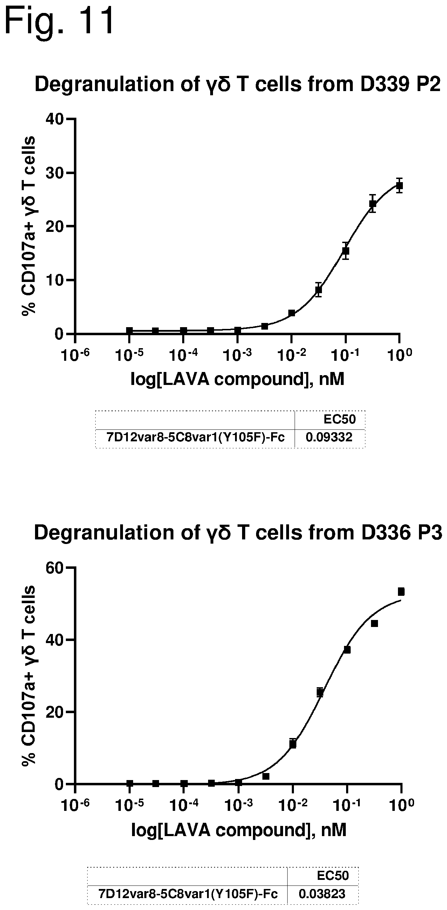

To investigate the potential of 7D12var8-5C8var1(Y105F)-Fc to activate Vγ9Vδ2 T cells, Vγ9Vδ2 T cells were first isolated and expanded as described before. Next, Vγ9Vδ2 T cells were then cultured together with A-431 tumor cells in a 1:1 E:T ratio in the presence of different concentrations of 7D12var8-5C8var1(Y105F)-Fc antibody and a PE-labelled anti-CD107a fluorescent antibody. After 24h, cells were harvested and stained with fluorescently labelled anti-Vy9 and anti-CD3 antibodies to differentiate Vγ9Vδ2 T cells from tumor cells. Using flow cytometry, the degree of CD107a expression on Vγ9Vδ2 T cells, reflecting target-dependent degranulation, was investigated. Figure 11 shows that with increasing concentrations of 7D12var8-5C8var1(Y105F)-Fc, Vγ9Vδ2 T cells were efficiently induced to degranulate dependent on A-431 cells. The EC50 for Vγ9Vδ2 T cell degranulation induced by 7D12var8-5C8var1(Y105F)-Fc lies in the picomolar range (~40-90 pM).

### Example 15| Antibody 7D12-5C8 induces T cell-mediated target cell cytotoxicity

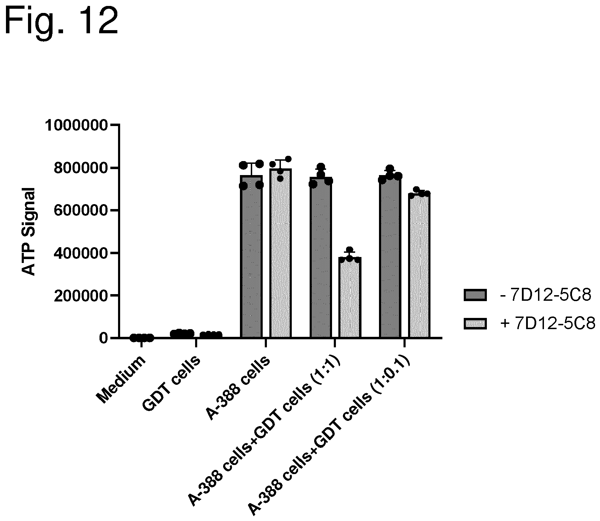

To investigate whether the bispecific VHH 7D12-5C8 was efficient in inducing Vγ9Vδ2 T cell-mediated cytotoxicity against target cells, the viability of the A-388 epidermoid tumor cell line (ATCC, CRL-7905) was assessed in a co-culture setting with Vγ9Vδ2 T cells and the bsVHH antibody fragment. In this assay, Vγ9Vδ2 T cells were used that were isolated from healthy PBMCs as described previously but subsequently frozen and stored at -150 °C. Frozen Vγ9Vδ2 T cells were thawed and rested overnight in IL-2 supplemented medium. A-388 tumor cells were seeded either alone or with rested Vγ9Vδ2 T cells in a 1:1 or 1:0.1 ratio, with or without 7D12-5C8(10 nM). As an additional control, Vγ9Vδ2 T cells were seeded alone with or without antibody 7D12-5C8 (10 nM). After 72h, the viability of cells was determined by the addition of ATP Lite (Perkin Elmer, 6016731) and readout of the luminescent signal with a microplate reader. Figure 12 shows the ATP-derived fluorescence signal, representing the metabolic active of- and thereby number of viable cells. At a 1:1 E:T ratio it can be observed that the antibody induces a reduction of viable cells of ca. 50% whereas untreated co-cultures of A-388 and Vγ9Vδ2 T cells are unaffected, underlining its potential to induce T cell-mediated cytotoxicity.

### Example 16| Tumor cell killing by 7D12-5C8 and 7D12-5C8var1(Y105S)-Fc activated Vγ9Vδ2 T cells

To investigate whether bsVHH 7D12-5C8 and antibody 7D12-5C8var1(Y105S)-Fc were able to induce Vγ9Vδ2 T cell-mediated cytotoxicity against patient-derived tumor cells, the viability of such tumor cells was assessed in a co-culture setting with Vγ9Vδ2 T cells and the antibodies. Various different types of tumor cells were tested.

Tissue samples (i.e. primary and metastatic tumor material derived from the colon, peritoneum and liver, head and neck squamous cell carcinoma (HNSCC) and non-small cell lung carcinoma (NSCLC)) were collected from cancer patients at the Amsterdam UMC (location VUmc) after written informed consent. Tissue was cut into small pieces with a surgical blade (size no. 22; Swann Morton Ltd), resuspended in dissociation medium composed of IMDM supplemented with 0.1% DNAse I, 0.14% Collagenase A, 5% FCS, 100 IU/ ml sodium penicillin, 100 µg/ml streptomycin sulfate and 2.0 mM L-glutamine, transferred to a sterile flask with stir bar and incubated on a magnetic stirrer for 45 minutes in a 37°C water bath. After incubation, cell suspensions were run through 100 µM cell strainers. Tumor was dissociated three times after which cells were washed for viable cell count with trypan blue exclusion.

Dissociated patient-derived tumor cells were incubated with healthy donor derived Vγ9Vδ2 T cells (1:1 E:T ratio) in the presence or absence of 50 nM 7D12-5C8 for 4 hours or 7D12-5C8var1(Y105S)-Fc or gp120-5C8var1(Y105S)-Fc for 24 hours.

Adhered cells were, if needed, detached after the culture period using trypsin-EDTA and resuspended in FACS buffer (PBS supplemented with 0.5% bovine serum albumin and 20 µg/ml NaN3), incubated with fluorescent dye labeled antibodies for 30 minutes at 4 degrees after which staining was measured by flow cytometry using an LSR Fortessa XL-20 (BD).

Living cells were identified using the life/death marker 7AAD in combination with 123counting eBeads^{™} according to manufacturer's instructions. Flow cytometry data were analyzed using Kaluza Analysis Version 1.3 (Beckman Coulter) and FlowJo Version 10.6.1 and 10.7.2 (Becton Dickinson).

7D12-5C8 and 7D12-5C8var1(Y105S)-Fc induced Vγ9Vδ2 T cell-mediated cytotoxicity of tumor cells was assessed by incubating expanded healthy donor derived Vγ9Vδ2 T cells with single cell suspensions of various malignant tumors (primary CRC, CRC metastases in peritoneum and liver, head and neck squamous cell carcinoma and non-small cell lung carcinoma).

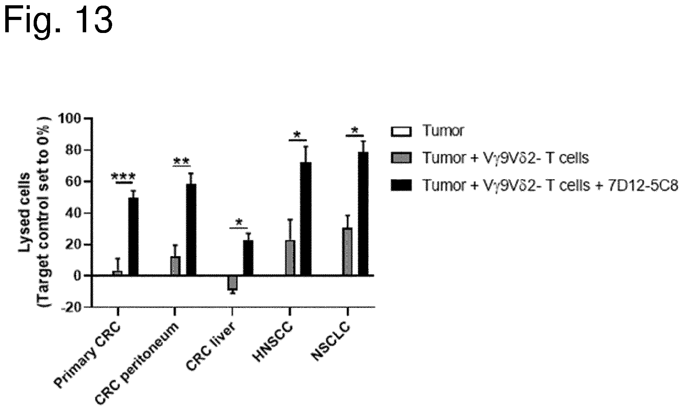

As illustrated in Figure 13, 7D12-5C8 induced substantial lysis of patient tumor cells by Vγ9Vδ2 T cells (mean % of lysis induced by 7D12-5C8: CRC primary 52.3% and p-value 0.0003, CRC peritoneum 46.0% and p-value 0.0052, CRC liver 31.8% and p-value 0.0360, head and neck squamous cell carcinoma 46.1% and p-value 0.0187 and non-small cell lung carcinoma 64.1% and p-value 0.0153).

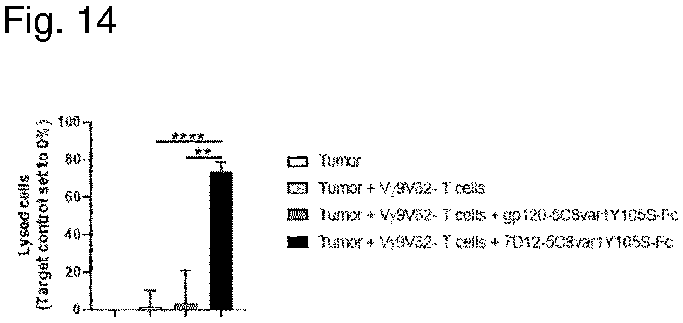

Furthermore, as shown in Figure 14, 7D12-5C8var1(Y105S)-Fc induced significant amount of lysis of patient tumor cells by Vγ9Vδ2 T cells (mean % of lysis induced by 7D12-5C8var1(Y105S)-Fc: 71.2% and p <0.0001 and 0.0012). The control compound gp120-5C8var1(Y105S)-Fc did not induce any measurable tumor cell lysis.

### Example 17| Design, production and purification of construct for non-human primate studies

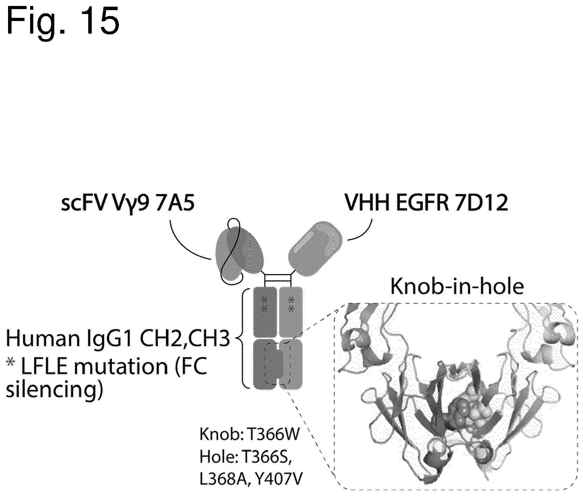

*For in vivo* studies in non-human primates, a construct with a binding domain that cross-reacts with the cynomolgous Vy9 TCR chain was generated (Figure 15). This binding domain was based on antibody 7A5, a TCR Vγ9-specific antibody (Janssen et al., J. Immunology 146(1) (1991), 35-39). Antibodies based on 7A5 have been found to bind cynomolgus Vγ9Vδ2 T cells (see Example 1 of WO2021052995). A bispecific Fc-containing antibody comprising 7A5 and anti-EGFR VHH 7D12var8 was constructed. The molecule contained a human IgG1 Fc tail that was engineered for hetero-dimerization using the knob-in-hole technology (KiH; Carter et al., 2001 Imm. Meth. 2001: 248, 7; Knob: T366W; Hole: T366S, L368A and Y407V). The Vy9 binding scFv of the 7A5 antibody was coupled to the 'knob' chain; the EGFR binding VHH 7D12var8 was cloned in-frame with the 'hole' chain of the KiH Fc pair. In addition, the upper hinge was engineered to 'AAASDKTHTCPPCP' to remove the cysteine (C220) that normally bridges to the CL and to introduce more flexibility by changing 'EPK' to 'AAA'. The N-terminal part of CH2 was engineered to abrogate Fc receptor (CD16, -32 and -64) interaction (silencing mutations L234F, L235E), while maintaining the FcRN binding. The resulting construct was termed 7A5-7D12var8-Fc.

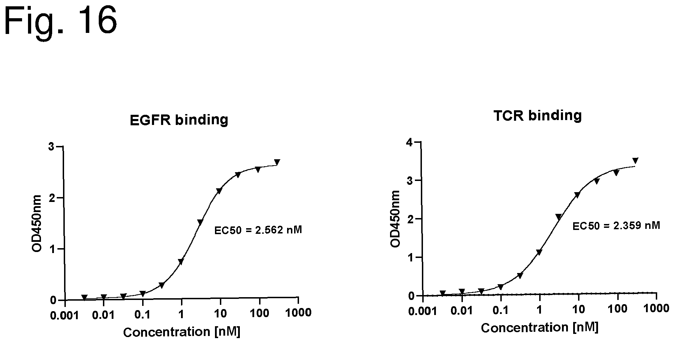

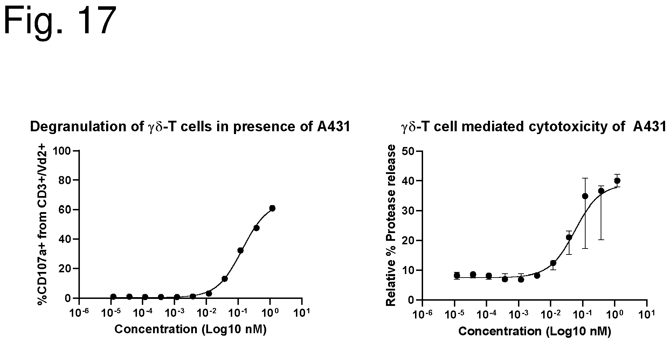

The molecule was produced by transient co-transfection of two plasmids encoding the two different chains in HEK293E cells and purified from the culture supernatant by protein-A affinity chromatography, followed by preparative size exclusion chromatography (Example 1). The molecule was shown to bind with roughly 3 nanomolar (nM) apparent affinity to either target using ELISA and recombinant forms of both antigens (Figure 16). The functionality of the molecule was demonstrated by showing that it caused target-dependent activation (CD107a expression) of in vitro expanded Vγ9Vδ2 T cells and subsequent T-cell mediated tumor cell lysis (Figure 17).

### Example 18| Bispecific antibody 7A5-7D12var8-Fc was well tolerated in an exploratory multiple-dose non-human primate (NHP: Cynomolgus monkey) study

In a multiple-dose exploratory NHP study, 7A5-7D12var8-Fc was administered to three female cynomolgus monkeys at 1mg/kg, 5mg/kg and 23mg/kg doses respectively. The antibody was given in half an hour infusions at 5mL/kg; 4 weekly infusions were administered. The first two dose groups (1 animal per dose) of 1 and 5mg/kg were dosed simultaneously and after three (weekly) doses, the third dose group (23mg/kg) received the first dose. Blood was regularly drawn from the animals for PK analyses, analyses of clinical chemistry parameters, measurements of cytokine levels and for analyses of blood cell subsets by flow cytometry. One day after the last dose was given, animals were euthanized and tissues were harvested and prepared for histopathological examination and for immunohistochemistry (IHC).

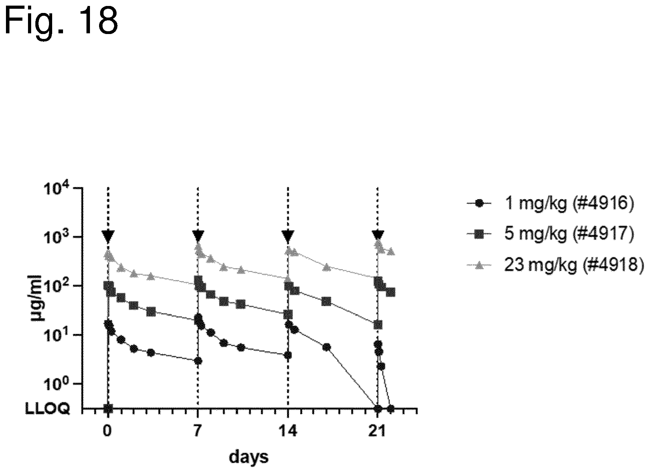

Pharmacokinetic analysis of 7A5-7D12var8-Fc concentrations in the blood of treated animals (measured in ELISA, Figure 18) revealed that the antibody displayed an IgG-like PK with a half-life that ranged between 84 and 127 hours. In the animal that was dosed at 1mg/kg, the antibody showed a shorter half-life after the third injection, which could be due to a possible anti-drug antibody (ADA) response in that animal.

Clearance values found were between 0.36 and 0.72 mL/h/kg and the volume of distribution was between 58.5 and 115.2 mL/kg. The systemic exposure increased dose-proportionally between 1 and 23 mg/kg. However, no accumulation was observed after repeated dosing.

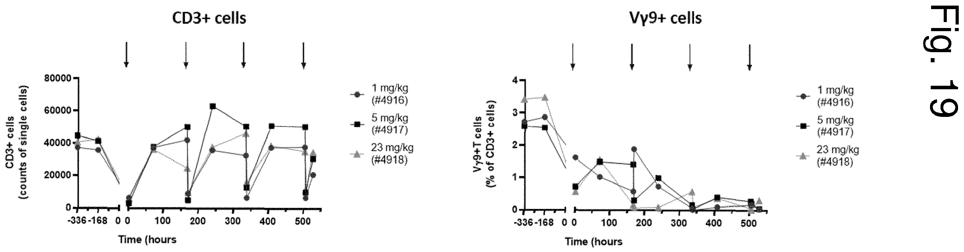

The compound could be detected by IHC in different tissues (lymph node, muscle, skin and colon); as expected, there was a dose-proportional intensity of compound staining in these tissues (data not shown). Flow cytometric analysis of blood cells showed several transient decreases in lymphocytes (Figure 19) that are often observed in these kinds of multiple-dose studies and that are procedurerelated. Figure 19 shows transient decreases in T cell counts at every time point 2 hours after dosing. However, the number of T cells returned to baseline levels two days after the injection.

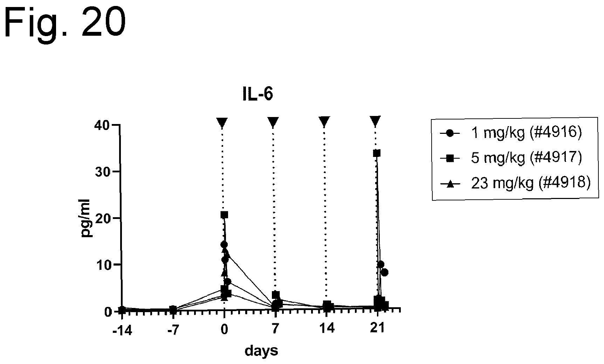

In contrast, Vγ9 positive T-cells decreased in numbers in peripheral blood and did not regain their former frequency. These cells stayed almost absent over the course of the study, demonstrating a specific pharmacodynamic effect of the compound. Measurements of cytokines in the blood of treated animas showed that the treatment caused very little cytokine release and that this was almost exclusively restricted to the first injection with compound. Figure 20 shows the levels of IL-6 measured as an example.

As a general conclusion, treatment of NHP with 7A5-7D12var8-Fc was very well tolerated and showed no clinical signs of toxicity. In addition, no macroscopic, nor microscopic aberrations of any of the examined organs were noted in histopathology (data now shown). In comparison: an anti-EGFR x CD3 BiTE was lethal for NHP at a dose of 31µg/kg/day of continuous infusion (Lutterbuese et al., Proc Natl Acad Sci U S A 2010: 107(28), 12605).

### Example 19| Stable formulations of 7D12var8-5C8var1(Y105F)-Fc

A stable CHO cell clone expressing 7D12var8-5C8var1(Y105F)-Fc was generated and antibody product was obtained through production in a bioreactor system. After harvest and purification the antibody product was formulated at concentrations of 1 mg/mL and 10 mg/mL using four types of formulation buffers shown table 6.

**Table 6| Overview of tested formulations**

| **Buffer** | **Formulation** |
|---|---|
| Buffer 1 | 10 mM Histidine + 280 mM Sucrose + 0.02% Polysorbate 80, pH 6.0 + 1 mM Methionine |
| Buffer 2 | 10 mM Histidine + 280 mM Sucrose + 0.02% Polysorbate 80, pH 6.0 |
| Buffer 3 | 10 mM Sodium acetate + 280 mM Sucrose + 0.02% Polysorbate 80, pH 5.5 + 1 mM Methionine |
| Buffer 4 | 10 mM Sodium acetate + 280 mM Sucrose + 0.02% Polysorbate 80, pH 5.5 |

Formulated samples were subjected to several storage conditions (defined below), for up to 6 weeks. Additionally, several stress tests were applied:
- Storage at 5°C ± 3°C
- Storage at -80°C ± 10°C
- Accelerated storage conditions at 25°C ± 2°C/ 60% relative humidity (RH) ± 5%
- Heat stress at 40°C ± 2°C/ 75% RH ± 5%
- Freeze/Thaw cycles: The samples were completely frozen to -80 ± 10°C.

Subsequently, five freeze/thaw cycles have been performed after allowing samples to completely thaw at room temperature (15 - 25°C).
- Agitation stress: Samples agitated at 240 rpm for 7 days at room temperature (EP15°C - 25°C).
- Oxidation: The samples were spiked with 0.01% (v/v) hydrogen peroxide (H₂O₂) and incubated for 24 hours at room temperature.
- Photostability: > 1.2 million lux hours and > 1000 watt hours/square meter of near ultra violet energy were applied at 25°C ± 2°C/ 60% RH ± 5%.

Initial (= t0) 5°C ± 3°C samples served as a reference. Samples were analyzed using the following analytical methods shown in Table 7.

**Table 7| Analytical procedures for formulation assessment**

| **Method** | **Assessment** |
|---|---|
| Clarity | Appearance |
| Color | Appearance |
| pH | pH |
| A₂₈₀ (UV/VIS) | Content |
| SE-HPLC | Integrity |
| Cation-exchange (CIEX)-HPLC | Integrity |
| Capillary electrophoresis sodium dodecyl sulfate (CE-SDS nonreduced) | Integrity |
| CE-SDS (reduced) | Integrity |
| Activity assay - PSMA binding ELISA | Potency |
| Peptide mapping | Identity |
| Differential scanning | Integrity |
| Liquid chromatography-mass spectrometry (LC-MS) (oxidation) | Integrity |

The results of the stability over time study were highly comparable for Clarity, Color, pH, A₂₈₀ (UV-VIS), SE-HPLC, CIEX-HPLC, CE-SDS and PSMA binding, Differential Scanning and Peptide Fingerprint for storage temperatures at -80°C ± 10°C, 5°C ± 3°C, 25°C ± 2°C / 60% RH ± 5% and 40°C ± 2°C / 75% RH ± 5%. The results of the measurements indicate that the product was stable with each of the chosen buffer conditions and only slight effects were observed over time. Furthermore, the results from the stress tests (freeze/thawing (five F/T cycles), photo-, agitation- and oxidation stress) showed that the product is stable under each of these conditions.

The main effects to differentiate between buffers were detected under heat stress and oxidation stress conditions. The CIEX-HPLC analyses of samples kept at 40°C ± 2°C / 75% RH ± 5% show a reduction of the main variant, coupled with an increase in acidic variants, for all tested buffers and product concentrations. However, the histidine-based formulation a show a lower reduction compared to the acetate-based formulation. In addition, LC-MS analysis revealed after oxidation stress showed that the addition of methionine effectively prevents oxidation of the product.

An overview of the most affected analytical results after temperature stability and oxidation stress is shown in Tables 8 and 9 for 1 mg antibody/mL and 10 mg antibody/mL formulation respectively.

In conclusion, all formulation buffers tested are considered suitable for 7D12var8-5C8var1(Y105F)-Fc. However, the antibody was most stable when formulated in buffer 1, by a small margin.

**Table 8| Analytical Results for temperature stability and oxidation stress stability of 7D12var8-5C8var1(Y105F)-Fc at 1 mg/mL in formulation.**

| **Buffer** | **Assay** | **Readout** | **1 mg/mL** | | | | | | | |
|---|---|---|---|---|---|---|---|---|---|---|
| | | | **Release** | **2-8C** | | **25C** | | **40C** | | **Oxidation** |
| | | | **0w** | **3w** | **6w** | **3w** | **6w** | **3w** | **6w** | **Stressed** |
| Acetate | CIEX-HPLC | Main (%) | NA | | | | | | | 70.4 |
| | | Acidic (%) | | | | | | | | 11.5 |
| | | Basic (%) | | | | | | | | 18.1 |
| | LC-MS | LAVA223 - M34^{∗} | 1.8 | NA | | | | | | 12.1 |
| | | LAVA224 - M34^{∗} | 1.8 | | | | | | | 3.2 |
| Acetate/ Methionine | General | Content (mg/mL) | 1.1 | 1.1 | 1.1 | 1.1 | 1.1 | 1.1 | 1.1 | NA |
| | | pH | 5.6 | 5.7 | 5.6 | 5.6 | 5.6 | 5.7 | 5.6 | |
| | SE-HPLC | Main peak (%) | 99.6 | 99.1 | 99.0 | 99.1 | 99.0 | 99.2 | 99.0 | NA |
| | | HMW (%) | 0.4 | 0.9 | 1.0 | 0.9 | 1.0 | 0.7 | 0.7 | |
| | | LMW (%) | 0.0 | 0.0 | 0.0 | 0.0 | 0.0 | 0.1 | 0.3 | |
| | CIEX-HPLC | Main (%) | 79.8 | 79.9 | 79.7 | 77.4 | 75.4 | 64.4 | 50.8 | 73.6 |
| | | Acidic (%) | 13.7 | 14.1 | 14.2 | 16.3 | 18.1 | 28.7 | 42.4 | 11.8 |
| | | Basic (%) | 6.4 | 6.0 | 6.1 | 6.4 | 6.5 | 6.8 | 6.8 | 14.6 |
| | LC-MS | LAVA223 - M34^{∗} | 1.8 | NA | | | | | | 11.8 |
| | | LAVA224 - M34^{∗} | 1.8 | | | | | | | 3.1 |
| Histidine | CIEX-HPLC | Main (%) | NA | | | | | | | 70.6 |
| | | Acidic (%) | | | | | | | | 11.3 |
| | | Basic (%) | | | | | | | | 18.1 |
| | LC-MS | LAVA223 - M34^{∗} | 1.8 | NA | | | | | | 11.8 |
| | | LAVA224 - M34^{∗} | 1.8 | | | | | | | 2.9 |
| Histidine/ Methionine | General | Content (mg/mL) | 1.0 | 1.0 | 1.1 | 1.1 | 1.1 | 1.1 | 1.1 | NA |
| | | pH | 5.9 | 6.0 | 5.9 | 6.0 | 5.9 | 6.0 | 5.9 | |
| | SE-HPLC | Main peak (%) | 99.7 | 99.3 | 99.2 | 99.2 | 99.2 | 99.2 | 99.1 | NA |
| | | HMW (%) | 0.3 | 0.7 | 0.8 | 0.8 | 0.8 | 0.6 | 0.6 | |
| | | LMW (%) | 0.0 | 0.0 | 0.0 | 0.0 | 0.0 | 0.1 | 0.3 | |
| | CIEX-HPLC | Main (%) | 80.4 | 80.2 | 80.3 | 78.0 | 76.5 | 65.7 | 54.6 | 74.4 |
| | | Acidic (%) | 13.6 | 14.2 | 14.3 | 16.2 | 17.7 | 27.6 | 38.9 | 11.8 |
| | | Basic (%) | 5.9 | 5.6 | 5.4 | 5.8 | 5.8 | 6.8 | 6.6 | 13.9 |
| | LC-MS | LAVA223 - M34 | 1.8 | NA | | | | | | 10.3 |
| | | LAVA224 - M34 | 1.8 | | | | | | | 2.8 |

| | | | | | | | | | | |
|---|---|---|---|---|---|---|---|---|---|---|
| * Oxidation levels in Histidine/Methionine buffer for reference at release | | | | | | | | | | |

**Table 9| Analytical Results for temperature stability and oxidation stress stability of 7D12var8-5C8var1(Y105F)-Fc at 10 mg/mL in formulation.**

| **Buffer** | **Assay** | **Readout** | **10 mg/mL** | | | | | | | |
|---|---|---|---|---|---|---|---|---|---|---|
| | | | **Release** | **2-8C** | | **25C** | | **40C** | | **Oxidation** |
| | | | **0w** | **3w** | **6w** | **3w** | **6w** | **3w** | **6w** | **Stressed** |
| Acetate | CIEX-HPLC | Main (%) | NA | | | | | | | 70.5 |
| | | Acidic (%) | | | | | | | | 11.3 |
| | | Basic (%) | | | | | | | | 18.2 |
| | LC-MS | LAVA223 - M34^{∗} | 1.8 | NA | | | | | | 11.6 |
| | | LAVA224 - M34^{∗} | 1.8 | | | | | | | 3.3 |
| Acetate/ Methionine | General | Content (mg/mL) | 10.6 | 10.6 | 10.6 | 10.7 | 10.6 | 10.8 | 10.7 | NA |
| | | pH | 5.6 | 5.6 | 5.6 | 5.6 | 5.6 | 5.6 | 5.6 | |
| | SE-HPLC | Main peak (%) | 99.5 | 99.4 | 99.4 | 99.4 | 99.3 | 98.9 | 98.3 | NA |
| | | HMW (%) | 0.5 | 0.6 | 0.6 | 0.6 | 0.7 | 1.0 | 1.3 | |
| | | LMW(%) | 0.0 | 0.0 | 0.0 | 0.0 | 0.0 | 0.1 | 0.3 | |
| | CIEX-HPlC | Main (%) | 80.2 | 79.9 | 79.9 | 76.1 | 75.3 | 63.7 | 50.0 | 73.3 |
| | | Acidic (%) | 13.6 | 14.0 | 14.0 | 16.1 | 17.9 | 28.5 | 42.2 | 11.7 |
| | | Basic (%) | 6.2 | 6.1 | 6.1 | 7.9 | 6.8 | 7.8 | 7.8 | 15.0 |
| | LC-MS | LAVA223 - M34^{∗} | 1.8 | NA | | | | | | 10.2 |
| | | LAVA224 - M34^{∗} | 1.8 | | | | | | | 2.9 |
| Histidine | CIEX-HPLC | Main (%) | NA | | | | | | | 70.8 |
| | | Acidic (%) | | | | | | | | 11.2 |
| | | Basic (%) | | | | | | | | 18.0 |
| | LC-MS | LAVA223 - M34^{∗} | 1.8 ' | NA | | | | | | 11.4 |
| | | LAVA224 - M34^{∗} | 1.8 | | | | | | | 3.0 |
| Histidine/ Methionine | General | Content (mg/mL) | 10.4 | 10.4 | 10.5 | 10.6 | 10.3 | 10.9 | 10.5 | NA |
| | | pH | 5.9 | 6.0 | 5.9 | 6.0 | 5.9 | 6.0 | 5.9 | |
| | SE-HPLC | Main peak (%) | 99.6 | 99.5 | 99.5 | 99.4 | 99.3 | 98.9 | 98.5 | NA |
| | | HMW (%) | 0.5 | 0.5 | 0.5 | 0.6 | 0.7 | 0.9 | 1.2 | |
| | | LMW (%) | 0.0 | 0.0 | 0.0 | 0.0 | 0.0 | 0.1 | 0.3 | |
| | CIEX-HPLC | Main (%) | 80.3 | 79.8 | 80.1 | 77.1 | 76.3 | 65.7 | 54.8 | 73.4 |
| | | Acidic (%) | 13.6 | 14.1 | 14.2 | 15.8 | 17.4 | 27.0 | 38.0 | 11.7 |
| | | Basic (%) | 6.1 | 6.1 | 5.7 | 7.1 | 6.2 | 7.3 | 7.1 | 14.9 |
| | LC-MS | LAVA223 - M34^{∗} | 1.8 | NA | | | | | | 10.5 |
| | | LAVA224 - M34^{∗} | 1.8 | | | | | | | 3.0 |

| | | | | | | | | | | |
|---|---|---|---|---|---|---|---|---|---|---|
| ^{∗} Oxidation levels in Histidine/Methionine buffer at 1 mg/mL for reference at release | | | | | | | | | | |

## Claims

1. A pharmaceutical composition comprising:
a. an antibody comprising a first antigen-binding region capable of binding to human Vδ2, wherein said first antigen-binding region comprises a CDR1 sequence as set forth in SEQ ID NO:1, a CDR2 sequence as set forth in SEQ ID NO:2 and a CDR3 sequence as set forth in SEQ ID NO:3,
b. 5-20 mM of a L-histidine buffer, wherein the composition has a pH between 5.5 and 6.5, or
5-20 mM of a sodium acetate buffer, wherein the composition has a pH between 5.0 and 6.0,
c. 250-350 mM sucrose,
d. 0.01% - 0.05% (w/v) polysorbate 80, and
e. 0-20 mM L-methionine.

2. The pharmaceutical composition according to claim 1, wherein the composition comprises 5-20 mM, such as 10 mM, of a L-histidine buffer, wherein the composition has a pH between 5.5 and 6.5.

3. The pharmaceutical composition according to any one of the preceding claims, wherein the composition comprises between 250 and 300 mM, such as 280 mM sucrose.

4. The pharmaceutical composition according to any one of the preceding claims, wherein the composition comprises between 0.01% and 0.03%, such as 0.02%, polysorbate 80.

5. The pharmaceutical composition according to any one of the preceding claims, wherein the composition comprises between 0.5 and 20 mM L-methionine, such as 1 mM of methionine.

6. The pharmaceutical composition according to any one of the preceding claims, wherein the composition comprises between 0.2 and 20 mg/mL, such as 1 or 10 mg/mL, of said antibody.

7. The pharmaceutical composition according to any one of the preceding claims, wherein
• X₁ in SEQ ID NO:1 is S (Ser) and X₂ in SEQ ID NO:3 is F (Phe), or
• X₁ in SEQ ID NO:1 is S (Ser) and X₂ in SEQ ID NO:3 is S (Ser).

8. The pharmaceutical composition according to any one of the preceding claims, wherein the first antigen-binding region is a single-domain antibody and wherein the first antigen-binding region preferably comprises or consists of:
• the sequence set forth in SEQ ID NO:4, or
• a sequence having at least 90%, such as at least 92%, e.g. at least 94%, such as at least 96%, e.g. at least 98% sequence identity to the sequence set forth in SEQ ID NO:4.

9. The pharmaceutical composition according to any one of the preceding claims, wherein the antibody further comprises a second antigen-binding region and wherein the second antigen-binding region preferably is a single-domain antibody.

10. The pharmaceutical composition according to claim 9, wherein the antibody is a bispecific antibody and comprises a second antigen-binding region capable of binding human EGFR and wherein the second antigen-binding region preferably comprises the CDR1 sequence set forth in SEQ ID NO:5, the CDR2 sequence set forth in SEQ ID NO:6 and the CDR3 sequence set forth in SEQ ID NO:7, and wherein, more preferably, the second antigen-binding region comprises or consists of
• the sequence set forth in SEQ ID NO:8, or
• a sequence having at least 90%, such as at least 92%, e.g. at least 94%, such as at least 96%, e.g. at least 98% sequence identity to the sequence set forth in SEQ ID NO:8.

11. The antibody according to any one of the preceding claims, wherein the antibody further comprises an Fc region, wherein the Fc region preferably is a heterodimer comprising two Fc polypeptides, wherein the first antigen-binding region is fused to the first Fc polypeptide and the second antigen-binding region is fused to the second Fc polypeptide and wherein the first and second Fc polypeptides comprise asymmetric amino acid mutations that favor the formation of heterodimers over the formation of homodimers, wherein preferably the CH3 regions of the Fc polypeptides comprise said asymmetric amino acid mutations, and wherein preferably the first Fc polypeptide comprises a T366W substitution and the second Fc polypeptide comprises T366S, L368A and Y407V substitutions, or vice versa, wherein the amino acid positions correspond to human IgG1 according to the EU numbering system.

12. The pharmaceutical composition according to any claim 11 or 12, wherein the first and second Fc polypeptides comprise a mutation at position 234 and/or 235, preferably the first and second Fc polypeptide comprise an L234F and an L235E substitution, wherein the amino acid positions correspond to human IgG1 according to the EU numbering system.

13. The pharmaceutical composition according to any one of claims 17 to 20, wherein the antibody comprises a second antigen-binding region and wherein the first antigen-binding region comprises the sequence set forth in SEQ ID NO:4, the second antigen-binding region comprises the sequence set forth in SEQ ID NO:8 and
- the first Fc polypeptide comprises the sequence set forth in SEQ ID NO:11 and the second Fc polypeptide comprises the sequence set forth in SEQ ID NO:12, or
- the first Fc polypeptide comprises the sequence set forth in SEQ ID NO:12 and the second Fc polypeptide comprises the sequence set forth in SEQ ID NO:11.

14. The pharmaceutical composition according to any one of the preceding claims, wherein the antibody comprises or consists of the sequences set forth in SEQ ID NO:16 and SEQ ID NO:17 or comprises or consists of the sequences set forth in SEQ ID NO:16 and SEQ ID NO:18.

15. The pharmaceutical composition according to any one of the preceding claims for use as a medicament, preferably for use in the treatment of cancer.
